# EUROPEAN PATENT SPECIFICATION

(11) **EP 0 533 825 B1**
(45) Date of publication and mention of the grant of the patent: **24.04.1996**
(21) Application number: 91912194.7
(22) Date of filing: 14.06.1991
(51) Int. Cl.: C07H 17/00, A61K 31/70

(54) **ETHER LIPID-NUCLEOSIDE COVALENT CONJUGATES**
KOVALENTE-THER LIPIDNUKLEOSID-KONJUGATE
CONJUGUES COVALENTS DE LIPIDES D'ETHER-NUCLEOSIDES

(30) Priority: 15.06.1990 US 539001
(43) Date of publication of application: 31.03.1993
(73) Proprietor: WAKE FOREST UNIVERSITY, Winston-Salem, NC 27103 (US); THE UNIVERSITY OF NORTH CAROLINA AT CHAPEL HILL, Chapel Hill, NC 27599-4100 (US)
(72) Inventor: PIANTADOSI, Claude, Chapel Hill, NC 27514 (US); MARASCO, Canio, J., Jr., Tonawanda, NY14150 (US); KUCERA, Louis, S., Pfafftown, NC 27040 (US)
(74) Representative: Stokes, Graeme Wilson
(86) International application number: US9104289
(87) International publication number: WO9119726

(56) References cited:
- EP-A- 0 350 287
- WO-A-90/00555
- US-A- 4 291 024
- US-A- 4 622 392
- US-A- 4 797 479
- US-A- 4 921 951
- JOURNAL OF MEDICINAL CHEMISTRY vol. 34, no. 4, April 1991, WASHINGTON US pages 1408 - 14 C.PIANTADOSI ET AL. 'Synthesis and Evaluation of Novel Ether Lipid Nucleoside Conjugates for Anti-HIV-1 Activity.'
- Journal of Medicinal Chemistry, Vol. 33, No. 5, issued 1990, CHUNG HONG et al., "Nucleoside Conjugate II. Synthesis and Antitumor Activity of 1-B-D-Acabinofuranosylcytosine and Cystine Conjugates in Thioether Lipids", pages 1380-1386, see entire document.
- AIDS Research and Human Retroviruses, Volume 6, No. 4, issued 1990, L.S. KUCERA et al., "Novel Membrane-Interactive Ether Analogs that Inhibit Infectious HIV-1 Production and Induce Defective Virus Formation", pages 491-501, see the entire document.

## Description

### Field of the Invention

The present invention relates to antiviral compounds in general, and particularly relates to covalent conjugates of ether lipids and antiviral nucleoside analogs, which conjugates have antiviral activity.

### Background of the Invention

The currently preferred treatment for combating human immunodeficiency virus type 1 (HIV-1) infections is by the administration of 3'-azido-3'-deoxythymidine, or AZT, to an afflicted subject. See, e.g., U.S. Patent No. 4,724,232 to Rideout et al.

C. Piantadosi et al., PCT Appln No. US89 04747 (published May 17, 1990), discloses a method of combating HIV-1 infections which comprises administering various ether lipid compounds in an amount effective to inhibit replication of the virus in infected cells. See also L. Kucera et al., Aids Research and Human Retroviruses 6, 491 (1990).

Various lipid derivatives of antiviral nucleosides, and the liposomal incorporation thereof, are disclosed in PCT Application Serial No. WO 90/00555 of K. Hostetler et al. See also K. Hostetler et al., J. Biol. Chem 265, 6112, 6113 Fig. 1 (1990).

U.S. Patent No. 4,291,024 to Turcotte concerns cytotoxic liponucleotide analogs, and U.S. Patent No. 4,921,951 to Shuto et al. discloses antineoplastic nucleoside-phospholipid conjugates.

In spite of prior efforts, there is an ongoing need for new ways to treat HIV-1 infections. The present invention is based on our continuing research in this area.

### Summary of the Invention

Disclosed herein are ether lipid-nucleoside covalent conjugates (or "lipid-nucleoside conjugates") of Formula (I) below and the salts thereof: 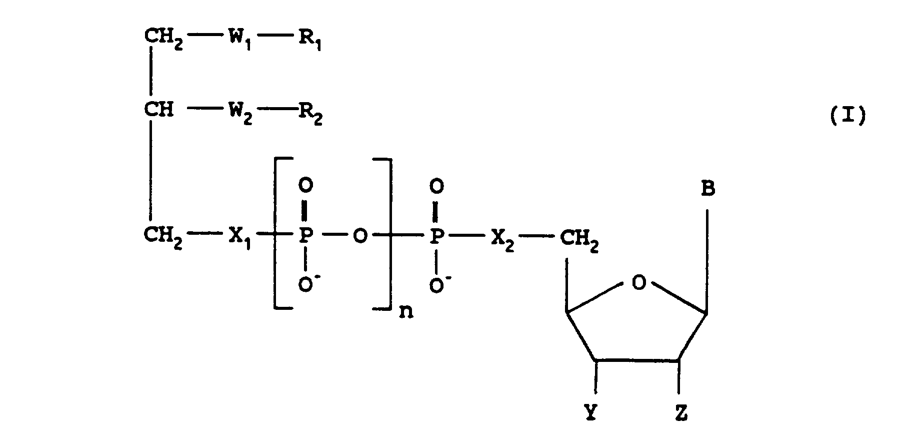 wherein:

R₁ is C10-C20 saturated or unsaturated alkyl containing not more than three double bonds. Preferably, R₁ is C16-C18 linear alkyl containing not more than one double bond.

R₂ is H or C1-C20 saturated or unsaturated alkyl containing not more than three double bonds. Preferably, R₂ is H or C1-C3 alkyl.

W₁ is S, O, NHC(=O), or NH. Preferably W₁ is NHC(=O).

W₂ is S, O, NHC(=O), OC(=O), NH, or a covalent bond. Preferably, W₂ is O.

n is zero or one.

X₁ and X₂ are each independently oxygen or a covalent bond, subject to the proviso that when n is zero, then at least either X₁ or X₂ is O.

Y is H, F, or N₃; Z is H or F; or Y and Z together are a covalent bond (i.e., form a didehydro). Preferably, Y is H or N₃; Z is H; or Y and Z together are a covalent bond. More preferably, Y is H or N₃ and Z is H.

B is a base such as adenine, thymine, cytosine, guanine, hypoxanthine, uracil, 5-fluoro-cytosine, 2-fluoro-adenine, 2-chloro-adenine, 2-bromo-adenine, and 2-amino-adenine.

Also disclosed herein are lipid-nucleoside conjugates of Formula (II) below and the salts thereof: 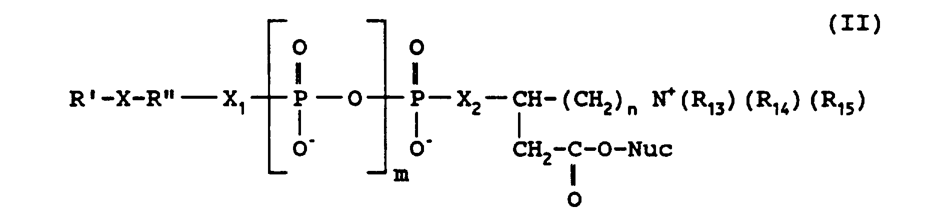 wherein:

X is S, O, NHC(=O), OC(=O), or NH, preferably NHC(=O).

R' is linear or branched, saturated or unsaturated C10-C20 alkyl containing not more than four double bonds, linear or branched, saturated or unsaturated C10-C20 acyl containing not more than four double bonds, phenyl, or naphthyl. More preferably, R' is C14-C20 linear saturated or unsaturated alkyl containing not more than three double bonds. Most preferably, R' is C16-C18 linear alkyl containing not more than one double bond.

R" is C5 to C6 cycloalkylene, or a straight-chained or branched, saturated or unsaturated aliphatic hydrocarbon chain containing 2-8 carbon atoms, which is unsubstituted or substituted one or more times by hydroxyl, phenyl, C1-C20 acyloxy, C1-C20 alkylthio, C1-C20 acylated amino, C1-C20 alkyl, or by C1-C20 alkoxy which is unsubstituted or is substituted by phenyl or C1-C5 alkoxy. Preferably, R" is C2-C4 linear alkyl which is unsubstituted or is substituted one or two times by hydroxyl, phenyl, C1-C20 acyloxy, C1-C20 alkylthio, C1-C20 acylated amino or by C1-C20 alkoxy which is unsubstituted or is substituted by phenyl or C1-C5 alkoxy. More preferably, R" is linear C2-C4 alkyl which is unsubstituted or substituted one or two times by hydroxyl, phenyl, C1-C20 acyloxy, C1-C20 alkylthio, C1-C20 acylated amino or by C1-C20 alkoxy which is unsubstituted or is substituted by phenyl or C1-C5 alkoxy.

m is zero or one.

X₁ and X₂ are each independently oxygen or a covalent bond, subject to the proviso that when m is zero, then at least either X₁ or X₂ is O.

n is 1 to 3. Preferably n is 1.

R₁₃, R₁₄, and R₁₅ are each independently either hydrogen or methyl, preferably methyl.

Nuc is: 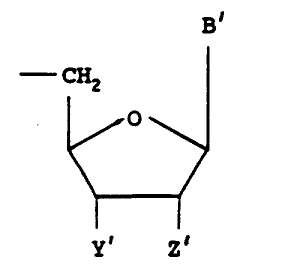 wherein:

Y' is H, F, or N₃; Z is H or F; or Y' and Z' together are a covalent bond (i.e., form a didehydro). Preferably, Y' is H or N₃; Z' is H; or Y' and Z' together are a covalent bond. More preferably, Y' is H or N₃ and Z' is H.

B' is a base such as adenine, thymine, cytosine, guanine, hypoxanthine, uracil, 5-fluoro-cytosine, 2-fluoro-adenine, 2-chloro-adenine, 2-bromo-adenine, and 2-amino-adenine.

A specific example of compounds of Formula (II) above are those of Formula (III) below and the salts thereof: 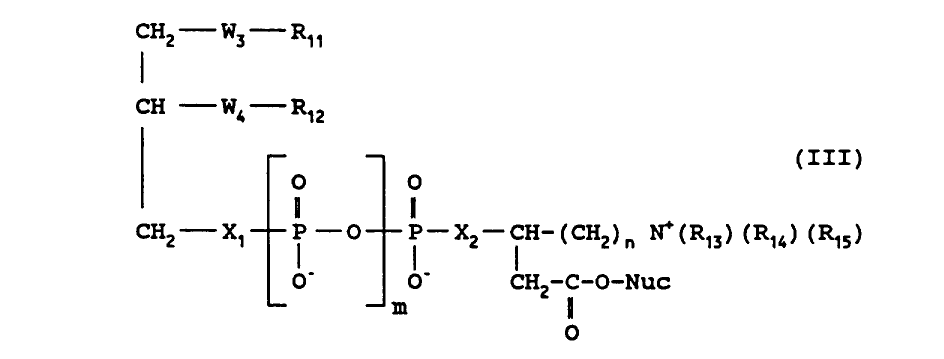 wherein:

R₁₁ is C10-C20 saturated or unsaturated alkyl containing not more than three double bonds. Preferably, R₁₁ is C16-C18 linear alkyl containing not more than one double bond.

R₁₂ is H or C1-C20 saturated or unsaturated alkyl containing not more than three double bonds. Preferably, R₁₂ is H or C1-C3 alkyl.

W₃ is S, O, NHC(=O), OC(=O), or NH. Preferably W₃ is NHC(=O).

W₄ is S, O, NHC(=O), OC(=O), NH, or a covalent bond. Preferably, W₄ is O.

m is zero or one.

X₁ and X₂ are each independently oxygen or a covalent bond, subject to the proviso that when m is zero, then at least either X₁ or X₂ is O.

n is 1 to 3. Preferably n is 1.

R₁₃, R₁₄, and R₁₅ are each independently either hydrogen or methyl, preferably methyl.

Nuc is as given in connection with Formula (II) above.

Also disclosed are pharmaceutical compositions comprising a lipid-nucleoside conjugate according to Formula I, II, or III above in a pharmaceutically acceptable carrier, wherein the lipid-nucleoside conjugate is included in the composition in an HIV-1 combating amount.

Also disclosed is the use of a lipid-nucleoside conjugate according to Formula I, II or III above to prepare a pharmaceutical composition or medicament for combating an HIV-1 infection in an afflicted subject.

The conjugates or salts of the present invention may be used in a method of combating HIV-1 infections in an afflicted subject comprising administering the subject an effective HIV-1 combating amount of a lipid-nucleoside conjugate according to Formula I, II or III above.

### Detailed Description of the Invention

Phospholipid-nucleoside conjugates of the present invention (e.g., Compounds A-D) may be prepared according to Scheme 1. The starting alcohols are synthesized as previously described. See M. Marx et al., J. Med. Chem. 31, 858 (1988); S. Morris-Natschke et al., J. Med. Chem. 29, 2114 (1986). 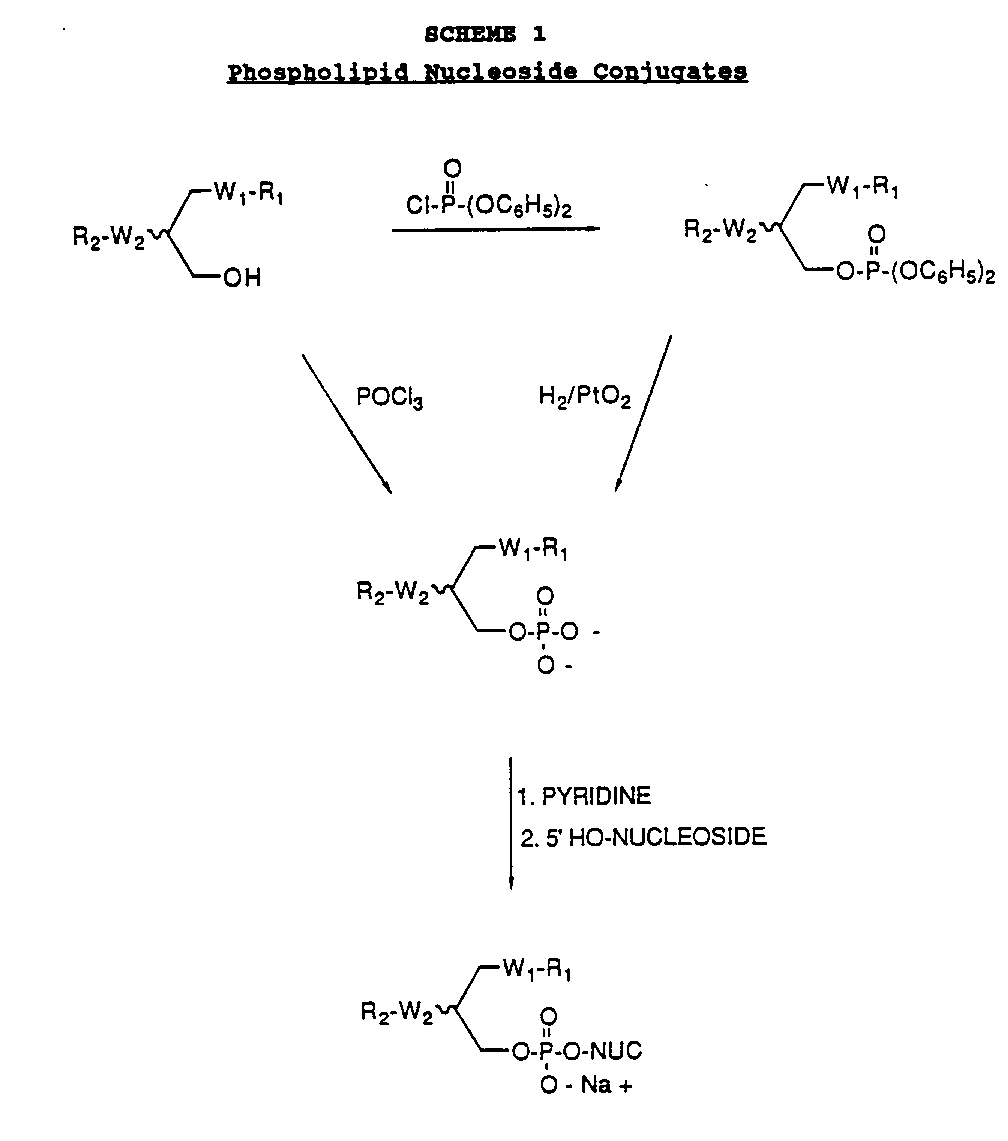 The amidoalkyl glycerol derivative is phosphorylated with diphenylchlorophosphate in pyridine to give the corresponding phosphate ester. See C. Piantadosi, J. Pharm. Sci. 62, 320 (1973). The phenyl groups are then removed via hydrogenolysis with PtO₂ to give the intermediate. The thio and oxygen ether derivatives are phosphorylated by an alternative procedure using phosphorus oxychloride and triethylamine or pyridine. See Ether Lipids: Biochemical and Biomedical Aspects, 403 (H. Mayold and F. Paltauf eds. 1983); C. Hong et al., J. Med. Chem. 29, 2038 (1986). The phosphatidic acid derivatives are then conjugated to the 5' hydroxyl of the appropriate nucleoside (NUC) via a dicyclohexylcarbodiimide (DCC) condensation, and subsequent conversion to the sodium salt gave the desired products. See E. Ryu et al., J. Med. Chem. 25, 1322 (1982).

The synthesis of the phosphonate analogue (e.g., Compound E) is shown in Scheme 2. 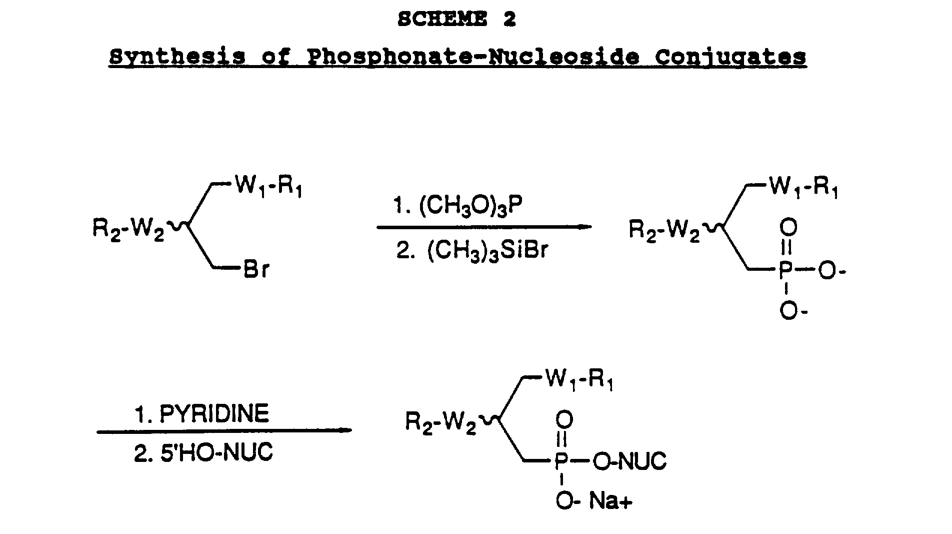 Starting with the appropriate bromopropane, see C. Marasco et al., J. Med. Chem. 33, 985 (1990), the halide is displaced with trimethylphosphite to afford the corresponding phosphonate. B. Arbuzov, Pure Appl. Chem.9, 307 (1964). The protective methyl groups are then cleaved with trimethylsilylbromide, see R. Bittman et al., Chem. Phys. Lipids 34, 201 (1984), to give the expected phosphonic acid. Condensation of the phosphonic acid intermediate with a nucleoside such as AZT is done in the usual manner to give product phosphonate.

The carnitine conjugates (e.g., Compound AA) are prepared according to Scheme 3. 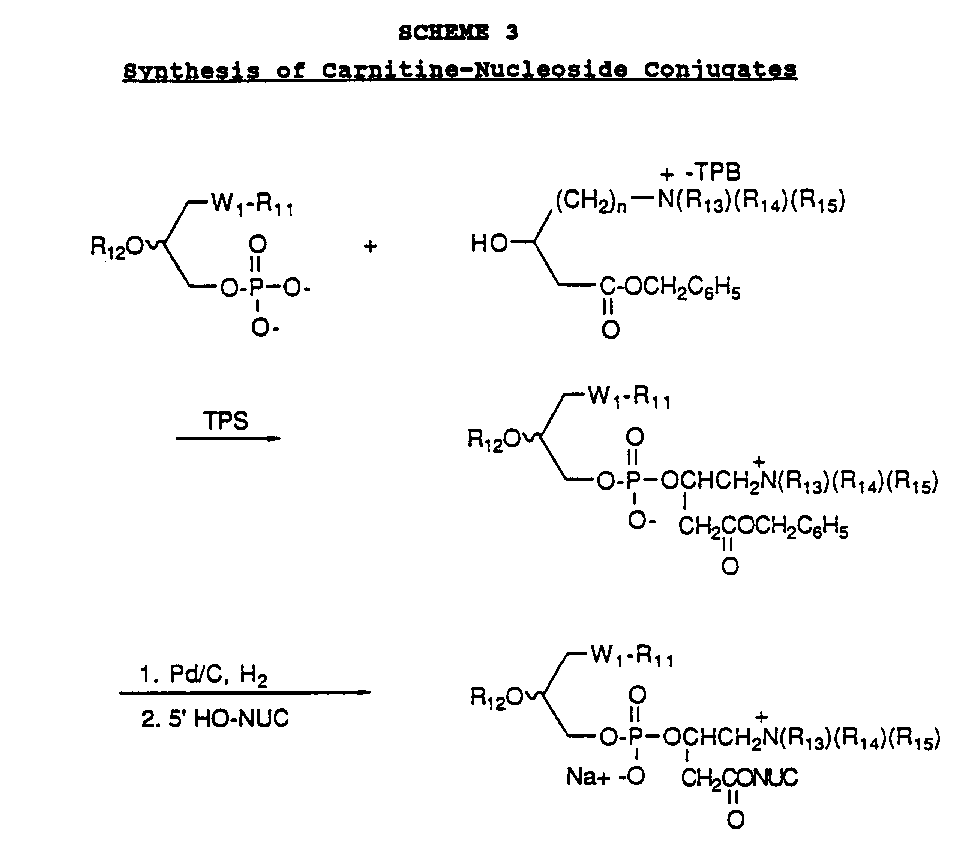 The condensation of the starting intermediate with the benzyl ester of carnitine as the tetraphenylborate salt is done via a 2,4,6-triisopropylbenzenesulfonylchloride (TPS) coupling to give a benzyl esterified carnitine. See U. Hintze and G. Gercken, Lipids 10, 20 (1974). The benzyl ester of the esterified intermediate is then cleaved by hydrogenolysis with Pd on activated carbon to yield the free carboxylic acid. Condensation of this intermediate with a nucleoside such as AZT is then performed in the usual manner to give the expected product as the sodium salt.

The pyrophosphate or phosphonophosphate conjugates are synthesized from the condensation of the appropriate dialkyl or amidoalkyl phosphatidic or phosphonic acid derivative with the appropriate 5'-monophosphomorpholidate nucleoside as the N,N'-dicyclohexylcarboxamidinium salt in pyridine. The phosphophosphonate conjugate is synthesized in an analogous manner from the appropriate dialkyl or amido alkyl phosphatidic acid congener and the appropriate 5'-phosphonomorpholidate nucleoside as the N,N'-dicyclohexylcarboxamidinium salt.

In case the compounds disclosed above have an asymmetric carbon atom, the present invention also concerns the enantiomeric forms. The resolution of the racemates into the enantiomeric forms can be done in the last step of the process, or in the appropriate preceding step, by known procedures, for example, by converting the racemate with an optically active reagent into a diasteriomeric pair and subsequent resolution thereof.

Exemplary antiviral nucleosides which may be covalently joined to the 5' carbon on the ribose ring to form lipid-nucleoside conjugates of the present invention include 3'-deoxythymidine; 3'-fluoro-3'-deoxythymidine; 2',3'-dideoxycytidine; 2',3'-dideoxy-5-fluoro-cytidine; 2',3'-dideoxyadenosine; 3'-azido-2',3'-dideoxyadenosine; 2'-fluoro-2',3'-dideoxyadenosine; 2',3'-dideoxy-2-fluoro-adenosine; 2',3'-dideoxy-2-chloro-adenosine; 2',3'-dideoxy-2-bromo-adenosine; 2',3'-dideoxy-2-amino-adenosine; 2',3'-dideoxyguanosine; 3'-azido-2',3'-dideoxyguanosine; 3'-azido-2',3'-dideoxyuridine; 2',3'-didehydro-2',3'-dideoxycytidine, and 2',3'-didehydro-2',3'-dideoxythymidine. See generally H. Mitsuya et al., Proc. Natl. Acad. Sci. USA 82, 7096 (1985); H. Mitsuya and S. Broder, Proc. Natl. Acad. Sci. USA 83, 1911 (1986); P. Herdewijn et al.; J. Med. Chem. 30, 1276 (1987); C-H. Kim et al., J. Med. Chem. 30, 862 (1987); V. Marquez et al., Biol. Chem. Pharm. 36, 2719 (1987); T. Haertle et al., J. Cellular Biochem. Suppl. 11D, 65 (1987); J. Balzarini et al., Biochem. Biophys. Res. Comm. 145 277 (1987); M. Baba et al., Biochem. Biophys. Res. Comm. 145, 1080 (1987); R. Schinazi et al., J. Cellular Biochem. Suppl. 11D, 74 (1987); Y. Hamamoto et al., Antimicrob. Agents and Chemother. 31, 907 (1987). Conjugates of 3'-Azido-3'-deoxythymidine are preferred.

The following compounds are illustrative of the compounds of Formula I above. These compounds may be prepared by the procedures described herein, or by variations thereof which will be apparent to those skilled in the art in light of the instant disclosure.
(A) 3'-azido-3'-deoxythymidine-5'-monophosphate-D,L-3-octadecanamido-2-ethoxypropane;
(B) 3'-azido-3'deoxythymidine-5'-monophosphate-D,L-3-hexadecyloxy-2-ethoxypropane;
(C) 3'azido-3'-deoxythymidine-5'-monophosphate-D,L-3-hexadecylthio-2-methoxypropane;
(D) 2',3'-dideoxyinosine-5'-monophosphate-D,L-3-octadecanamido-2-ethoxypropane;
(E) 3'-Azido-3'-deoxythymidine-5'-phosphono-D,L-3-hexadecyloxy-2-methoxypropane;
(F) 3'-Azido-3'-deoxythymidine-5'-monophosphate-D,L-3-octadecanamido-2-hexadecyloxypropane;
(G) 3'-Azido-3'-deoxythymidine-5'-monophosphate-D,L-3-octadecanamido-2-palmitoylpropane;
(H) 3'-Azido-3-deoxythymidine-5'-diphosphate-D,L-3-octadecanamido-2-ethoxypropane;
(I) 3'-Azido-3'-deoxythymidine-5'-phospho-1-phosphono-D,L-3-octadecanamido-2-ethoxypropane;
(J) 3-Azido-3'-deoxythymidine-5'phosphono-1-phospho-D,L-3-octadecanamido-2-ethoxypropane;
(K) 3'-deoxythymidine-5'-monophosphate-D,L-3-octadecanamido-2-ethoxypropane;
(L) 3'-fluoro-3'-deoxythymidine-5'-monophosphate-D,L-3-hexadecyloxy-2-ethoxypropane;
(M) 2',3'-dideoxycytidine-5'-monophosphate-D, L-3-hexadecylthio-2-methoxypropane;
(N) 2',3'-dideoxy-5-fluoro-cytidine-5'-monophosphate-D,L-3-octadecanamido-2-ethoxypropane;
(O) 2',3'-dideoxyadenosine-5'-phosphono-D,L-3-hexadecyloxy-2-methoxypropane;
(P) 3'-Azido-2',3'-dideoxyadenosine-5'-monophosphate-D,L-3-octadecanamido-2-hexadecanoylpropane;
(Q) 3'-Azido-2',3'-dideoxyadenosine-5'-monophosphate-D,L-3-octadecanamido-2-palmitoylpropane;
(R) 2'-fluoro-2',3'-dideoxyadenosine-5'-diphosphate-D,L-3-octadecanamido-2-ethoxypropane;
(S) 2',3'-didehydro-2',3'-dideoxycytidine-5'-phospho-1-phosphono-D,L-3-octadecanamido-2-ethoxypropane;
(T) 3'-Azido-2',3'-dideoxyuridine-5'-phosphono-1-phospho-D,L-3-octadecanamido-2-ethoxypropane;
(U) 2',3'-Dideoxy-2-fluoro-adenosine-5'-monophosphate-D,L-3-octadecanamido-2-ethylpropane;
(V) 2',3'-Dideoxy-2-chloro-adenosine-5'-monophosphate-D,L-3-hexadecyloxy-2-ethylpropane;
(W) 2',3'-Dideoxy-2-amino-adenosine-5'-monophosphate-D,L-3-hexadecylthio-2-hexadecylthiopropane;
(X) 2',3'-Dideoxy-2-bromo-adenosine-5'-monophosphate-D,L-3-octadecanamido-2-octadecanamidopropane;
(Y) 2',3'-Dideoxyguanosine-5'-phosphono-D,L-3-hexadecylamino-2-hexadecylaminopropane;
(Z) 3'-Azido-2',3'-dideoxyguanosine-5'-monophosphate-D,L-3-octadecanamino-2-hexadecyloxypropane; and
(A') 3'-Azido-3'-deoxythymidine-5'-diphosphate-D,L-3-hexadecyloxy-2-ethoxypropane.

The following compounds are illustrative of the-compounds of Formula II above. These compounds may likewise be prepared by the procedures described herein, or variations thereof which will be apparent to those skilled in the art in light of the present disclosure.
(AA) 3'-Azido-3'-deoxythymidine-5'-butyrate-γ-N,N,N-trimethyl-ammonium-β-(1-phospho-2-ethoxy-3-hexadecyloxypropane); and
(BB) 3'-Azido-3-deoxythymidine-5'-butyrate-γ-N,N,N-trimethyl-ammonium-β-(1-phospho-2-ethoxy-3-octadecanamidopropane).

The lipid-nucleoside conjugates disclosed herein can be prepared in the form of their pharmaceutically acceptable salts or their non-pharphaceutically acceptable salts. The non-pharmaceutically acceptable salts are useful as intermediates for the preparation of a pharmaceutically acceptable salt. Pharmaceutically acceptable salts are salts that retain the desired biological activity of the parent compound and do not impart undesired toxicological effects. Examples of such salts are (a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, polygalacturonic acid, and the like; and (b) salts formed from elemental anions such as chlorine, bromine, and iodine.

The lipid-nucleoside conjugates described above can be combined with an inert pharmaceutical carrier to provide a pharmaceutical composition for enteral or parenteral administration. The compounds described above being the active ingredient in these compositions, they should be included in an amount effective to accomplish the intended treatment. For the preparation of these compositions, use can be made of pharmaceutical carriers adapted for all conventional forms of administration, for example, tablets, capsules, dragees, syrups, solutions, suspensions and the like. As injection medium, it is preferred to use water which contains the additives usual in the case of injection solutions, such as stabilizing agents, solubilizing agents and/or buffers. Additives of this kind include, for example, human serum albumin and synthetic analogs thereof, tartrate and citrate buffers, ethanol, complex formers (such as ethylenediamine-tetraacetic acid and the non-toxic salts thereof) and high molecular weight polymers (such as liquid polyethylene oxide) for viscosity regulation. Liquid carrier materials for injection solutions must be sterile and are preferably placed into ampules. Solid carrier materials include, for example, starch, lactose, mannitol, methylcellulose, talc, highly dispersed silicic acids, high molecular weight fatty acids (such as stearic acid), gelatine, agar-agar, calcium phosphate, magnesium stearate, animal and vegetable fats and solid high molecular weight polymers (such as polyethylene glycols). Compositions suitable for oral administration can, if desired, contain flavoring and/or sweetening agents.

A method of combating human immunodeficiency virus Type 1 (HIV-1) infection in an afflicted subject comprises administering to the subject a lipid-nucleoside conjugate as described herein in an amount effective to inhibit replication of infectious virus in the subject. Likewise, a method of combating human immunodeficiency virus Type 1 (HIV-1) infection of cells comprises administering to the cells a lipid-nucleoside conjugate as described herein in an amount effective to inhibit replication of the virus in the cells. Administration of the lipid-nucleoside conjugate to an afflicted subject can be carried out by any suitable means, such as by intravenous administration, intraperitoneal administration, subcutaneous administration, and oral administration.

The dosage of lipid-nucleoside conjugate to be administered depends upon a variety of factors, such as mode of administration, species, age, and subject condition. Usually, the dosage to be administered is from about .05 to about 100 milligrams per kilogram of body weight, more preferably between about .1 and about 75 milligrams per kilogram of body weight, and most preferably between about .5 and about 50 milligrams per kilogram of body weight.

In the Examples below, proton nuclear magnetic reasonance spectra were recorded in CDCl₃ on either a BRUKER 300-MH_{z} or a VARIAN 400-MH_{z} spectrometer. Chemical shifts are reported in parts per million relative to internal tetramethylsilane. Infrared spectra were recorded on a Perkin-Elmer 1320 spectrometer as thin films. Melting points were determined on a Thomas-Hoover capillary melting point apparatus and are uncorrected. Microanalyses were performed by Atlantic Microlab Inc. Mass spectral data was obtained from a VG7OS Mass spectrometer. All reactions were performed under a positive pressure of dry nitrogen with dry solvents. Tetrahydrofuran (THF) was distilled from Na and benzophenone, dichloromethane (DCM) from phosphorus pentoxide, triethylamine (Et₃N) from KOH, and pyridine was stored over KOH. Chromatographic purification was performed using silica gel 60 (230-400 mesh). Thin layer chromatographic plates were visualized by iodine vapor, molybdenum phosphate spray, and charring following sulfuric acid spray.

### EXAMPLE 1

**(±)-3-Octadecanamido-2-ethoxypropyldiphenylphosphate.** To a three-neck round-bottom flask equipped with a magnetic stir bar, nitrogen inlet and reflux condenser was added a solution of (0.7 mL, 3.39 mmol) diphenylchlorophosphate in 10 mL anhydrous ether. The solution was cooled to 4°C, and a solution of the starting amidoalkyl glycerol¹ (1.0 g, 2.6 mmol) in 15 mL of pyridine and 5 mL of ether was then added. The solution was warmed to room temperature, and then heated to 52°C for 3 h. After cooling to room temperature, the reaction mixture was diluted with 50 mL of ether, extracted twice with 25 mL portions of distilled water, once with 25 mL of cold 0.5 N HCl, and once with 25 mL of distilled water. The ether layer was dried over sodium sulfate, filtered, and concentrated *in vacuo* to give a pale yellow oil. Purification by silica gel chromatography (discontinuous gradient of hexane:ethyl acetate 10:1 to 1:1 as eluent) gave 961 mg of pure product (60.1%). ¹H-NMR (CDCl₃):δ 0.87 (t, 3 H, terminal methyl), 1.1-1.3 [m, 31 H, (CH₂)₁₄, CH₃CH₂O], 1.55 (m, 2 H, NH-C-CH₂CH₂), 2.15 (t, 2 H, NH-C-CH₂), 3.3-3.6 (m, 5H, CH₃CH₂OCHCH₂NH), 4.25 (m, 2 H, CH₂OP), 5.9 (t, 1 H, NH), 7.15-7.35 [m, 10 H, (OC₆H₅)₂].

### EXAMPLE 2

**(±)-3-Octadenanamido-2-ethoxypropylphospatidic acid.** Into a Parr hydrogenation bottle was placed a solution of 500 mg of (±)-3-Octadecanamido-2-ethoxypropyldiphenylphosphate prepared according to Example 1 above in 100 mL of absolute ethanol. To the solution was added 69 mg of PtO₂, before placement onto the hydrogenation apparatus. The reaction mixture was placed under 14.5 psi of hydrogen, and shaken at room temperature. After 100 mins, 6 psi had been consumed, and TLC (CHCl₃:MeOH:H₂O, 70:35:4) indicated the absence of starting material. The reaction mixture was suction filtered through Celite, and the ethanol removed *in vacuo*. The resulting oil was taken up in 25 mL of pyridine, concentrated *in vacuo,* and dried under vacuum to give 352 mg (93.3%) of pure product as a fine powder. ¹H-NMR (CDCl₃):δ 0.87 (t, 3 H, terminal methyl), 1.1-1.3 [m, 31 H, (CH₂)₁₄, CH₃CH₂O], 1.55 (m, 2 H, NH-C-CH₂CH₂), 2.25 (t, 2 H, NH-C-CH₂), 3.3-3.75 (m, 5 H, CH₃CH₂OCHCH₂NH), 4.15 (m, 2 H, CH₂OP), 6.7 (t, 1 H, NH).

### EXAMPLE 3

**(±)-3-Hexadecyloxy-2-ethoxypropylphosphatidic acid.** To a three-neck round-bottom flask equipped with a magnetic stir bar, nitrogen inlet, and reflux condenser was added a solution of phosphorus oxychloride (0.62 mL, 6.6 mmol) in 5mL of THF. The solution was cooled to 0°C, and a solution of the starting dialkylglycerol (2.0 g, 5.8 mmol), and pyridine (1.4 mL, 17.3 mmol) in 15 mL of THF were added. The reaction mixture was maintained at 0°C for 3 h, and then 10 mL of 10% sodium bicarbonate was added. The mixture was stirred an additional 20 min, and poured into 30 mL of ice water. The solution was acidified by the dropwise addition of 2 N HCl, and then extracted twice with 30 mL portions of ether. The ether layer was dried over sodium sulfate, filtered, concentrated *in vacuo,* taken up in 100 mL of pyridine, concentrated, and dried under vacuum to give 1.5 g (46%) of product as a waxy solid. ¹H-NMR (CDCl₃):δ 0.87 (t, 3 H, terminal methyl), 1.1-1.3 [m, 29 H, (CH₂)₁₃, CH₃CH₂O], 1.4 (m, 2 H, OCH₂CH₂), 3.4-3.7 (m, 7 H, CH₃CH₂OCHCH₂OCH₂), 3.85 (m, 2 H, CH₂OP).

### EXAMPLE 4

**(±)-3-Hexadecylthio-2-methoxypropylphosphatidic acid.** To a three-neck round-bottom flask equipped with a magnetic stir bar, nitrogen inlet, and relux condenser was added a solution of phosphorus oxychloride (0.6 mL, 7 mmol) in 1 mL of hexane. The solution was cooled to 0°C, and a solution of triethylamine (1 mL, 10 mmol) in 1 mL of hexane was added dropwise. The starting thioalkyl glycerol (1.6 g, 5 mmol) was azeotropically dried with toluene, and the volume reduced to 10 mL. This was then added dropwise to the POCl₃/Et₃N solution, and stirred overnight at room temperature. One mL of water was added to the reaction mixture and stirred for 1 h. The reaction mixture was diluted with 20 mL of water, and extracted twice with 25 mL portions of ether. The organic layers were collected, dried over sodium sulfate, filtered, and concentrated *in vacuo.* The resulting oil was taken up into 50 mL of pyridine, heated to 50°C for 2 h, and concentrated *in vacuo.* Silica gel chromatography (CHCl₃:MeOH:NH₄OH, 70:35:1 to 70:35:7 as eluent) gave 535 mg of product. ¹H-NMR (CDCl₃):δ 0.87 (t, 3 H, terminal methyl), 1.2 [bs, 26 H, (CH₂)₁₃], 1.4 (m, 2 H, SCH₂CH₂), 2.4 (t, 2 H SCH₂CH₂), 2.5 (m, 2H, CHCH₂S), 3.4-3.7 (m, 4 H, CH₃OCHCH₂S), 4.0 (bm, 2 H CH₂OP).

### EXAMPLE 5

**3'-Azido-3'-deoxythymidine-5'-monophosphate-D,L-3-octadecanamido-2-ethoxypropane (Compound A).** Into a 25 mL round-bottom flask were placed (±)-3-Octadenanamido-2-ethoxypropylphospatidic acid (100 mg, 0.22 mmol) and AZT (43 mg, 0.16 mmol). The two reactants were then azeotropically dried by the *in vacuo* removal of 3 mL of pyridine three times. To this slurry dicyclohexylcarbodiimide (220 mg, 1.07 mmol) was added, and once again the reactants were azeotropically dried four times with 3mL portions of pyridine. The solution was then diluted to a final volume of 3 mL, the round bottom flask stoppered, and placed in a desiccator for 4 days. One g of water was added to the reaction mixture, and stirred at room temperature for 4 h. The solvents were removed *in vacuo,* and the resulting wax purified by silica gel chromatography (gradient of CHCl₃:MeOH, 15:1 to 2:1 as eluent) to give pure product. The product was dissolved in 11 mL of CHCl₃:MeOH:H₂O (4:6:1), placed in a round bottom flask, and stirred with 1.5 g of Whatman Pre-Swollen Microgranular Cation (Na+) Exchange Carboxymethyl Cellulose Resin for 1 h. The resin was filtered, and the filtrate concentrated *in vacuo* to give 32 mg of product as the sodium salt (21%). ¹H-NMR (CDCl₃):δ 0.87 (t, 3 H, terminal methyl), 1.1-1.3 [m, 31 H, (CH₂)₁₄,CH₃CH₂O], 1.55 (m, 2 H, NH-C-CH₂CH₂), 1.8 (s, 3 H, Thymidine CH₃), 2.1 (t, 2 H, NH-C-CH₂), 2.2 (m, 2 H, Thymidine 2' CH₂), 3.2-3.5 (m, 5 H, CH₃CH₂OCHCH₂NH), 3.75 (m, 2 H, CH₂OP), 3.85 (m, 1 H, Thymidine 4' CH), 3.95 (m, 2 H, Thymidine 5' CH₂), 4.35 (m, 1 H, Thymidine 3' CH), 6.1 (m, 1 H, Thymidine 1' CH), 6.95 (t, 1 H, NH), 7.4 (s, 1 H, Thymidine C₆ proton), 11.3 (bs, 1 H, diimide NH). FAB Mass Spectrum (M + 2Na)⁺; Theorectical 759.3795, Observed 759.3839 (2.0 ppm).

### EXAMPLE 6

**3'-Azido-3'-deoxythymidine-5'-monophosphate-D,L-3-hexadecyloxy-2-ethoxypropane (Compound B).** This analogue was made in analogous manner to that of 3'-Azido-3'-deoxythymidine-5'-monophosphate-D,L-3-octadecanamido-2-ethoxypropoane from 110 mg of (±)-3-Hexadecyloxy-2-ethoxypropylphosphatidic acid (0.26 mmol), 50 mg of AZT (0.19 mmol), and 250 mg of DCC (1.24 mmol) to give 37 mg of pure product (28%). ¹H-NMR (CDCl₃):δ 0.87 (t, 3 H, terminal methyl), 1.1-1.3 [m, 29 H, (CH₂)₁₃, CH₃CH₂O], 1.5 (m, 2 H, OCH₂CH₂), 1.8 (s, 3 H, Thymidine CH₃), 2.25 (m, 2 H, Thymidine 2' CH₂), 3.2-3.5 (m, 7 H, CH₃CH₂OCHCH₂OCH₂), 3.8 (m, 2 H, CH₂OP), 3.9 (m, 1 H, Thymidine 4' CH), 3.95 (m, 2 H, Thymidine 5' CH₂), 4.35 (m, 1 H, Thymidine 3' CH), 6.1 (m, 1 H, Thymidine 1' CH), 7.4 (s, 1 H, Thymidine C₆ proton), 11.3 (bs, 1 H, diimide NH). FAB Mass Spectrum (MH +Na)⁺; Theoretical 696.3713, Observed 696.3681 (4.6 ppm).

### EXAMPLE 7

**3'-Azido-3'-deoxythymidine-5'-monophosphate-D,L-3-hexadecylthio-2-methoxypropane (Compound C).** This analogue was made in analogous manner to that of 3'-Azido-3'-deoxythymidine-5'-monophosphate-D,L-3-octadecanamido-2-ethoxypropane (from 87 mg of (±)-3-hexadecylthio-2-ethoxypropyl phosphatidic acid (0.20 mmol), 43 mg of AZT (0.16 mmol), and 227 mg of DCC (1.1 mmol) to give 32 mg of pure product (23%). ¹H-NMR (CDCl₃):δ 0.87 (t, 3 H, terminal methyl), 1.1-1.3 [m, 26 H, (CH₂)₁₃], 1.45 (m, 2 H, SCH₂CH₂), 1.8 (s, 3 H, Thymidine CH₃), 2.25 (m, 2 H, Thymidine 2' CH₂), 2.4 (t, 2 H, S-CH₂), 2.6 (d, 2 H, CH₂-S), 3.3 (s, 3 H, CH₃O), 3.5 (m, 1 H, CH₃OCH), 3.9-4.1 (m, 5 H, CH₂OP, Thymidine 4' CH, 5' CH₂), 4.4 (m, 1 H, Thymidine 3' CH), 6.1 (m, 1 H, Thymidine 1' CH), 7.4 (s, 1 H, Thymidine C₆ proton), 11.3 (bs, 1 H, diimide NH). FAB Mass Spectrum (MH +Na)⁺ ; Theoretical 698.3328, Observed 698.3344 (2.2ppm).

### EXAMPLE 8

**2',3'-dideoxyinosine-5'-monophosphate-D,L-3-octadecanamido-2-ethoxypropane (Compound D).** This analogue was made in analogous manner to that of 3'-Azido-3'-deoxythymidine-5'-monophosphate-D,L-3-octadecanamido-2-ethoxypropane from 92 mg of (±)-3-Octadenanamido-2-ethoxypropyl phospatidic acid (0.20 mmol), 35 mg of DD1 (0.15 mmol), and 200 mg of DCC (1.0 mmol) to give 23 mg of pure product (22%). ¹H-NMR (CDCl₃);δ 0.87 (t, 3 H, terminal methyl), 1.1-1.3 [m, 31 H, (CH₂)₁₄, CH₃CH₂O], 1.55 (m, 2 H, NH-C-CH₂CH₂), 1.7 (m, 2 H Inosine 2' CH₂), 2.1 (m, 4 H, NH-C-CH₂, Inosine 3' CH₂), 3.1-4.1 (m, 10 H, CH₃CH₂OCHCH₂NH, CH₂OP, Inosine 4' CH, 5' CH₂), 6.1 (m, 1 H, Inosine 1' CH), 6.95 (t, 1 H, NH), 7.4 (s, 1 H, Inosine C₈ proton), 11.3 (bs, 1 H, diimide NH). FAB Mass Spectrum (M + 2Na)⁺; Theorectical 728.3739, Observed 728.3738 (0.2 ppm).

### EXAMPLE 9

**(±)-3-Hexadecyloxy-2-methoxypropyl dimethylphosphonate.** Into a three-neck round-bottom flask equipped with a magnetic stir bar, nitrogen inlet, and reflux condenser was placed a solution of the starting dialkyl halide, (954 mg, 2.4 mmol) in trimethylphosphite (4.987 g, 30.2 mmol). The solution was heated to 120°C for 90 h with continuous stirring. The reaction mixture was cooled to room temperature, reduced *in vacuo,* and purified by silica gel chromatography (gradient of petroleum ether:ether, 10:1 to 1:1 as eluent) to give 741 mg of product (80%) as a yellow viscous oil. ¹H-NMR (CDCl₃):δ 0.87 (t, 3 H, terminal methyl), 1.1-1.3 [m, 26 H, (CH₂)₁₃], 1.57 (m, 2 H, OCH₂CH₂), 2.08 (m, 2 H, CH₂-P), 3.4-3.6 (m, 7 H, CH₃OCHCH₂OCH₂), 3.75 [m, 7 H, CH₃OCH, P(OCH₃)₂].

### EXAMPLE 10

**(±)-3-Hexadecyloxy-2-methoxypropyl phosphonic acid.** To a three-neck round-bottom flask equipped with a magnetic stir bar, nitrogen inlet, and reflux condenser was added a solution of 6 (740 mg, 1.92 mmol) in 10 mL of alcohol-free chloroform. To this solution bromotrimethylsilane (1.6 g, 10.6 mmol) was added dropwise. After 1 h the solvents were removed *in vacuo,* and the resulting oil taken up in 25 mL of THF:H₂O (8:2), and stirred overnight at room temperature. The solvents were removed *in vacuo,* and the residue recrystallized from ether:acetonitrile (1:5) to give 577 mg of pure product (85%) as a white solid (MP 59-61°C). The product was taken up into 50 mL of pyridine, the pyridine removed *in vacuo,* and then dried under vacuum. ¹H-NMR (CDCl₃):δ 0.87 (t, 3 H, terminal methyl), 1.1-1.3 [m, 26 H, (CH₂)₁₃], 1.57 (m, 2 H, OCH₂CH₂), 2.12 (m, 2 H, CH₂-P), 3.4-3.6 (m, 7 H, CH₃OCHCH₂OCH₂), 3.75 (m, 1 H, CH₃OCH).

### EXAMPLE 11

**3'-Azido-3'-deoxythymidine-5'-phosphono-D,L-3-hexadecyloxy-2-methoxypropane (Compound E)**. This analogue was made in analogous manner to that of 3'-Azido-3'-deoxythymidine-5'-monophosphate-D,L-3-octadecanamido-2-ethoxypropoane from 100 mg of (±)-3-Hexadecyloxy-2-O-methoxypropyl phosphonic acid (0.26 mmol), 50 mg of AZT (0.19 mmol), and 250 mg of DCC (1.24 mmol) to give 29 mg of pure product (23%). ¹H-NMR (CDCl₃):δ 0.87 (t, 3 H, terminal methyl), 1.1-1.3 [m, 29 H, (CH₂)₁₃, CH₃CH₂O] 1.5 (m, 2 H, OCH₂CH₂), 1.8 (s, 3 H, Thymidine CH₃), 2.25 (m, 2 H, Thymidine 2'CH₂), 3.2-3.5 (m, 7 H, CH₃CH₂OCHCH₂OCH₂), 3.7 (m, 2 H, CH₂P), 3.9 (m, 1 H, Thymidine 4' CH), 3.95 (m, 2 H, Thymidine 5' CH₂), 4.4 (m, 1 H, Thymidine 3' CH), 6.1 (m, 1 H, Thymidine 1' CH), 7.4 (s, 1 H, Thymidine C₆ proton), 11.3 (bs, 1 H, diimide NH). FAB Mass Spectrum (M + Na)⁺; Theorectical 688.3426, Observed 688.3437 (1.6 ppm).

### EXAMPLE 12

**(±)-3-Hexadecyloxy-2-ethoxypropylphosphosphocarnitine benzyl ester.** To a three-neck round-bottom flask equipped with a magnetic stir bar, nitrogen inlet, and reflux condenser a solution (243 mg, 0.45 mmol) of (±)-3-hexadecyloxy-2-ethoxypropylphospatidic acid in 10 mL of pyridine was added. To this solution 778 mg (1.36 mmol) of the benzyl ester of carnitine as the tetraphenylborate salt⁹, 412 mg (1.36 mmol) of 2,4,6-triisopropylbenzenesulfoyl chloride, and an additional 15 mL of pyridine were added. The reaction mixture was stirred continuously overnight at room temperature. To the reaction mixture 2.5 mL of distilled water was added, and stirring was continued for 1 h. The solvents were removed *in vacuo,* and the pale pink oil extracted three times with 30 mL portions of ether. The extract was cooled to 0°C for 4 h, filtered, and concentrated *in vacuo.* The resultant yellow oil was purified by silica gel chromatography (gradient CHCl₃:MeOH, 10:1 to 1:1) to give 180 mg of pure product (56%). ¹H-NMR (CDCl₃): δ 0.87 (t, 3 H, terminal methyl), 1.1-1.4 [m, 29 H, (CH₂)₁₃, CH₃CH₂O], 1.5 (m, 2 H, OCH₂CH₂), 2.7 (m, 2H, P-O-CHCH₂COO-), 3.3-4.3 [m, 18 H, CH₃CH₂OCHCH₂OCH₂, CH₂N(CH₃)₃], 3.85 (m, 3 H, CH₂OPOCH), 5.1 (m, 2 H, OCH₂C₆H₅), 7.35 (bs, 5 H, C₆H₅).

### EXAMPLE 13

**(±)-3-Hexadecyloxy-2-ethoxypropylphosphosphocarnitine.** This analogue was made in similar manner to that of (±)-3-Octadenanamido-2-ethoxypropyl phospatidic acid from 110 mg of (±)-3-Hexadecyloxy-2-ethoxypropyl phosphosphocarnitine benzyl ester and a catalytic amount of Pd/C to give 88 mg of product 93%). ¹H-NMR (CDCl₃):δ 0.87 (t, 3 H, terminal methyl), 1.1-1.4 [m, 29 H, (CH₂)₁₃, CH₃CH₂O], 1.5 (m, 2 H, OCH₂CH₂), 2.7 [m, 2 H, CH₂N(CH₃)₃], 3.3-3.7 [m, 18 H, CH₃CH₂OCHCH₂OCH₂, P-O-CHCH₂-COO-, N(CH₃)₃], 3.95 (m, 3 H, CH₂OPOCH).

### EXAMPLE 14

**3'-Azido-3'-deoxythymidine-5'-α-carboxyphosphocholine-D,L-3-hexadecyloxy-2-ethoxypropane (Compound AA).** This analogue was made in analogous manner to that of 3'-Azido-3'-deoxythymidine-5'-monophosphate-D,L-3-octadecanamido-2-ethoxypropane from 75 mg of (±)-3-Hexadecyloxy-2-ethoxypropyl phosphocarnitine (0.12 mmol), 32 mg of AZT (0.12 mmol), and 160 mg of DCC (0.8 mmol) to give 41 mg of pure product. ¹H-NMR (CDCl₃):δ 0.87 (t, 3 H, terminal methyl), 1.1-1.3 [m, 29 H, (CH₂)₁₃, CH₃CH₂O], 1.5 (m, 2 H, OCH₂CH₂), 1.8 (s, 3 H, Thymidine CH₃), 2.25 (m, 2 H, Thymidine 2' CH₂), 2.7 [m, 2 H, CH₂N(CH₃)₃], 3.2-4.0 [m, 24 H, CH₃CH₂OCHCH₂OCH₂, CH₂OPOCHCH₂COO, N(CH₃)₃, Thymidine 4' CH, and 5' CH₂], 4.35 (m, 1 H, Thymidine 3' CH), 6.1 (m, 1 H, Thymidine 1' CH), 7.4 (s, 1 H, Thymidine C₆ proton), 11.3 (bs, 1 H, diimide NH). FAB Mass Spectrum (MH)⁺; Theoretical 817.4840, Observed 817.4867, 3.2 ppm.

### EXAMPLE 15

**Anti-HIV1 Activity of Lipid-Nucleoside Conjugates.** The inhibitory effects of lipid-nucleoside conjugates on the replication of human immunodeficiency virus type 1 (HIV-1) virus in cells was examined by the plaque assay procedure of L. Kucera et al., Aids Research and Human Retroviruses 6, 491 (1990). In brief, CEM-SS cell monolayers were infected with HIV-1. Infected cells were overlaid with RPMI-1640 plus 10% FBS supplemented with different concentrations of inhibitor. AZT and dideoxyinosine (DDI) were used as positive controls. Plaques were counted at five days after infection. In this assay HIV-1 syncytial plaques are seen as large, multicellular foci (10 to 25 nuclei/syncytium) that appear either brown and granular or clear. Silnce the number of HIV-1 syncytial plaques correlates with reverse transcriptase (RT) and p24 core antigen activity in the HIV-1 infected cell overlay fluids, the syncytial plaque assay can be used to quantify the amount of infectious virus. Reverse transcriptase activity was assayed according to a described procedure (B. J. Poeisz et al., Proc. Natl. Acad. Sci. (U.S.A.) 77, 7415 (1980)). The activity of p24 core antigen induced by HIV-1 infection of CEM-SS cells was measured spectrophotometrically using the commercial Coulter EIA.

The results (Table 1) showed that all the lipid-nucleoside conjugates tested have an IC₅₀ against HIV-1 syncytial plaque formation ranging from 0.02 to 1.56 µM. The conjugates IC₅₀ for cell cytotoxicity ranged from 25.2 to >100 µM. Of interest are data indicating that the differential selectivity for the conjugates ranged from >64 to 1793 compared to 1400 for AZT and >59 for DDI. The highest differential selectivity (1793) was obtained with the amidoalkyl lipid-AZT conjugate. The increased differential selectivity of the amidoalkyl lipid-AZT conjugate over AZT alone (1400) is due to about a ten-fold decrease in cell cytotoxicity of the amidoalkyl lipid-AZT conjugate (IC₅₀ = 53.8 µM) compared to AZT (IC₅₀ = 5.6 µM). The differential selectivity of the amidoalkyl lipid-AZT is about ten-fold higher than the phosphatidyl AZT prodrug reported by K. Hostetler et al., J. Biol. Chem. 265, 6112 (1990).

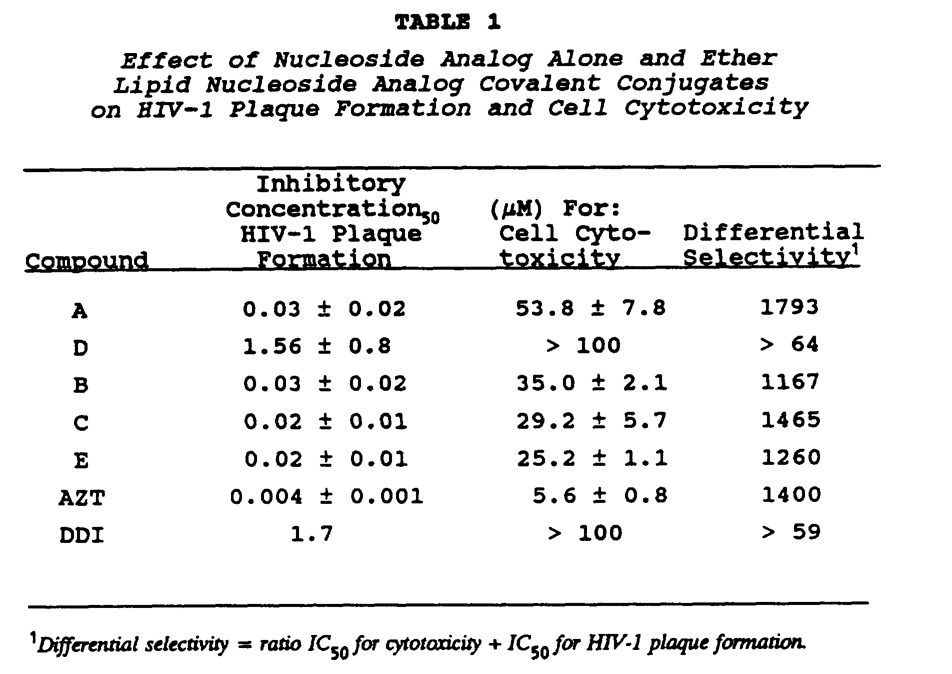

### EXAMPLE 16

**Anti-HIV-1 Activity of Lipid-Nucleoside Conjugates Over Time.** The effect of Compound A on HIV-1 acutely infected H9 cells and persistently infected H9IIIB cells was evaluated by measuring reverse transcriptase (RT) and infectious virus production in supernatant fluids harvested at various times (days) after HIV-1 infection and continuous treatment with compound A. The results (Table 2) indicated that Compound A caused a marked inhibition of both reverse transcriptase (RT) and infectious HIV-1 production in continuously treated and acutely infected H9 cells. In persistently infected H9IIIB cells, Compound A had little effect on RT activity but a marked inhibition of infectious HIV-1 production (Table 2). The best interpretation of these results is that Compound A inhibits reverse transcription and integration of provirus DNA and infectious virus production in HIV-1 acutely infected cells. In persistently infected cells that already have integrated provirus DNA before treatment, Compound A markedly inhibits infectious virus production.

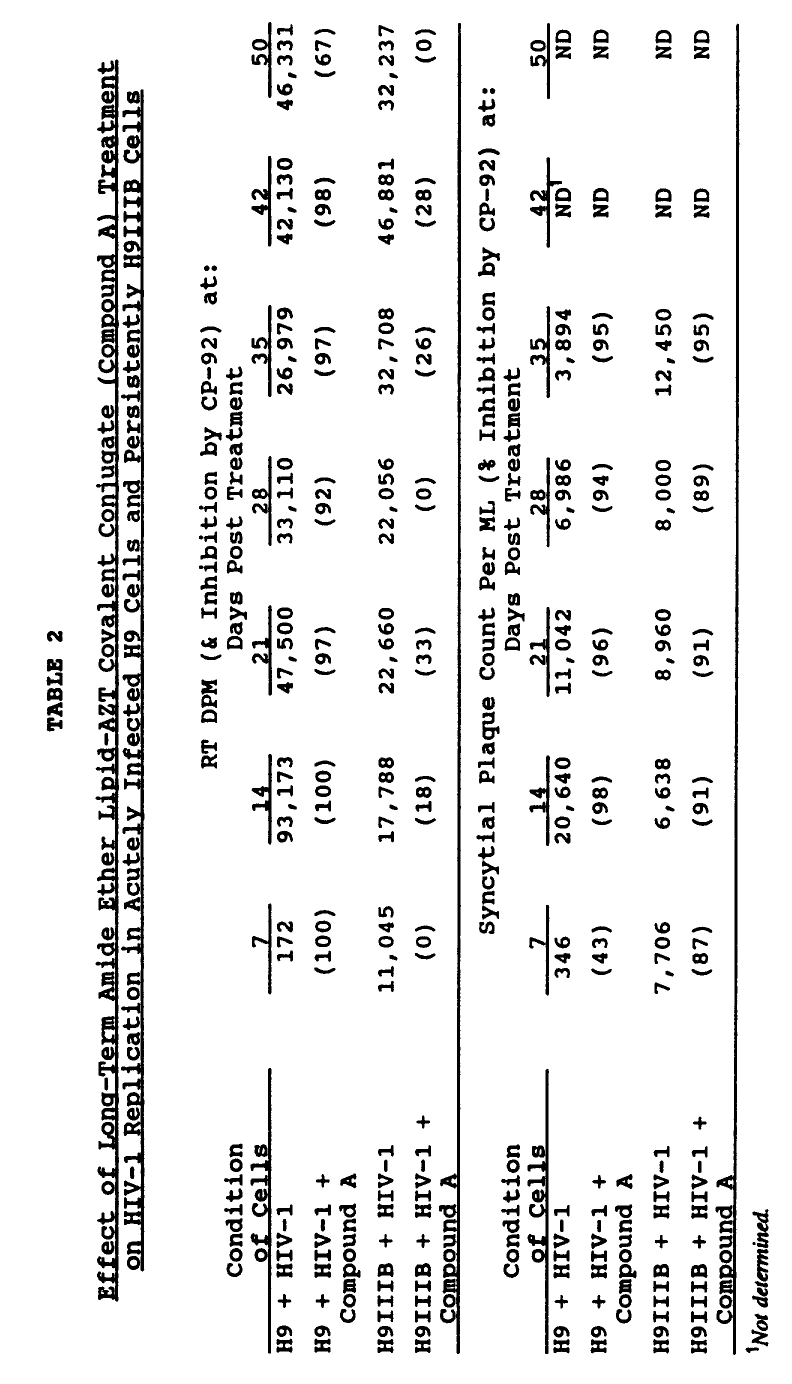

### EXAMPLE 17

**Anti-HIV1 Activity of Lipid-Nucleoside Conjugates in Monocyte/Macrophages.**
Monocyte/macrophages represent a major reservoir of HIV-1 in the infected human host. See L. Epstein et al., AIDS Res. 14, 447 (1984). However, these cells tend to be resistent to dideoxynucleoside prodrugs due to low levels of kinases needed to activate the prodrugs. See C. Perno et al., J. Exp. Med. 168, 1111 (1984). To test the compounds of the present invention in these cells, we treated HIV-1 persistently infected monocyte/macrophage (U1) cells with AZT and Compound A and measured the effect on HIV-1 replication. The results (Table 3) indicate that the compounds did not significantly inhibit HIV-1 induced RT and p24 core antigen production. As expected, AZT alone caused only 13% inhibition of infectious HIV-1 production. However, Compound A inhibited infectious HIV-1 production by 33%.

**TABLE 3**

| | | | |
|---|---|---|---|
| *Effect of AZT and Lipid-Nucleoside Conjugate on HIV-1 Induced RT, p24 Core Antigen Synthesis and Infectious Virus Production in Persistently Infected Monocyte/Macrophage Cells* | | | |

| Compound | (RT DPM) | Percent of Control (p24 Core Ag) | (PFU) |
|---|---|---|---|
| Control | (79,328) 100 | (94) 100 | (750) 100 |
| + AZT | 90 | 101 | 87 |
| + Compound A | 121 | 84 | 67 |

### EXAMPLE 18

**(±)-3-Octadecanamido-2-ethoxypropyl phosphocarnitine benzyl ester.** This compound was made in a similar manner to that of (±)-3-hexadecyloxy-2-ethoxypropyl phosphocarnitine benzyl ester from 206 mg of 3-octadecanamido-2-ethoxypropyl phosphatidic acid, 766 mg of the benzyl ester of carnitine as the tetraphenylborate salt, and 400 mg of triisopropylbenzenesulfonyl chloride giving 74 mg of product (32%). ¹H-NMR (CDCl₃): δ 0.87 (t, 3 H, terminal methyl), 1.1 (t, 3H, OCH₂CH₃), 1.2-1.4 [m, 28 H, (CH₂)₁₄], 1.5 (m, 2 H, NHCOCH₂CH₂), 2.1 (t, NHCOCH₂), 2.7 (m, 2H, P-O-CHCH₂-COO-), 3.0-3.7 [m, 16 H, CH₃CH₂OCHCH₂NHCO, CH₂N(CH₃)₃], 3.9 (m, 3 H, CH₂OPOCH), 5.1 (m, 2 H, OCH₂C₆H₅), 6.85 and 6.95 (m, 1H, NH diastereomers), 7.35 (bs, 5 H, C₆H₅).

### EXAMPLE 19

**(±)-3-Octadecanamido-2-ethoxypropyl phosphocarnitine.** The above benzyl ester (74 mg) was hydrogenated at 15 psi using a catalytic amount of Pd/C to give 53 mg of product (83%). ¹H-NMR (CDCl₃): δ 0.87 (t, 3 H, terminal methyl), 1.1 (t, 3H, OCH₂CH₃), 1.2-1.4 [m, 28 H, (CH₂)₁₄], 1.5 (m, 2 H, NHCOCH₂CH₂), 2.1 (t, NHCOCH₂), 2.7 (m, 2 H, P-O-CHCH₂-COO-), 3.3-4.0 [m, 18 H, CH₃CH₂OCHCH₂NHCO, CH₂N(CH₃)₃, CH₂OPOCH), 5.1 (m, 1H, OPOCH), 6.9 (m, 1H, NH).

### EXAMPLE 20

**3'-Azido-3'-deoxythymidine-5'-α-carboxyphosphocholine-D,L-3-octadecanamido-2-ethoxypropane** (compound BB). This analogue was made in analogous manner to that of 3'-azido-3'-deoxythymidine-5'-monophosphate-D,L-3-octadecanamido-2-ethoxypropane from 48 mg of (±)-1-octadecanamido-2-ethoxypropyl phosphocarnitine, 17 mg of AZT, 11 mg of N,N-dimethylaminopyridine, and 87 mg of DCC to give 8 mg of pure product (15% yield). ¹H-NMR (CDCl₃): δ 0.87 (t, 3 H, terminal methyl), 1.1 (t, 3H, CH₃CH₂O), 1.2-1.4 [m, 28 H, (CH₂)₁₄], 1.5 (m, 2 H, NHCOCH₂CH₂), 1.8 (s, 3 H, Thymidine CH₃), 2.1 (t, 2H, NHCOCH₂), 2.2-2.7 (m, 4H, Thymidine 2' CH₂, P-O-CHCH₂-COO-), 3.2-4.1 [m, 22 H, CH₃CH₂OCHCH₂NHCO, CH₂OPO, CH₂N(CH₃)₃, Thymidine 4' CH, and 5' CH₂], 4.6-5.5 (m, 3H, Thymidine 3' CH, OPOCH, Thymidine 1' CH), 6.9 (m, 2H, Thymidine C6 proton, NH).

### EXAMPLE 21

**3'-Azido-3'-deoxythymidine-5'-diphosphate-D,L-3-octadecanamido-2-ethoxypropane** (Compound H). 3-Octadecanamido-2-ethoxypropyl phosphatidic acid (36 mg, 0.08 mmol) was azeotropically dried with pyridine (3 ml) three times. AZT 5'-monophosphate morpholidate (25 mg, 0.06 mmol) was added and the drying repeated four times. An additional 3 ml of pyridine was added and the reaction allowed to continue for 96 hours at room temperature under nitrogen. After removal of the pyridine under vacuum, the resulting oil was chromatographed on 2 g of silica gel eluting with chloroform:methanol (65:35) to chloroform:methanol:water (65:35:1 to 65:35:4). Impure fractions were collected and rechromatographed using as eluent, chloroform to chloroform:methanol (9:1 to 2:1) to chloroform:methanol:water (2:1:0.1 to 2:1:0.4). The resulting pure product was dissolved in chloroform:methanol:water (4:6:1) and converted to the sodium salt by stirring twice with Na⁺ ion-exchange resin (1.5 g) for one hour. ¹H-NMR (CD₃OD): δ 0.8 (t, 3 H, terminal methyl), 1.1 (t, 3H, CH₃CH₂O), 1.2-1.4 [m, 28 H, (CH₂)₁₄], 1.55 (m, 2 H, NHCOCH₂CH₂), 1.8 (s, 3 H, Thymidine CH₃), 2.2 (t, 2H, NHCOCH₂), 2.2-2.5 (m, 2H, Thymidine 2' CH₂), 3.3-3.8 [m, 16 H, CH₃CH₂OCHCH₂NHCO, CH₂N(CH₃)₃) 3.9-4.2 (m, 5H, CH₂OPO, Thymidine 4' CH, and 5' CH₂] 4.6 (m, 1H, Thymidine 3' CH), 6.25 (m, 1H, Thymidine 1' CH), 7.8 (m, 2H, Thymidine C6 proton, NH). FAB Mass Spectrum (MH+2Na)⁺; Theoretical 839.3461, Observed 839.3463, 0.2 ppm.

### EXAMPLE 22

**3'-Azido-3'-deoxythymidine-5'-diphosphate-D,L-3-hexadecyloxy-2-ethoxypropane (compound A').** This compound is prepared in essentially the same manner as the compounds described above, except that 3-hexadecyloxy-2-ethoxypropyl phosphatidic acid is used as the starting material.

### EXAMPLE 23

**Anti-HIV1 Activity of Lipid-Nucleoside Conjugates.** CEM-SS cells were seeded (50,000 cells/ml RPMI-1640 growth medium) as a monolayer in 96-well dishes, innoculated with 50 to 100 plaque forming units of HIV-1 and overlaid with serial dilutions of lipid-nucleoside conjugate in RPMI-1640 growth medium. Plaques were counted after five days incubation at 37°C to determine the 50% inhibitory concentration.

To determine the IC₅₀ for cell growth, CEM-SS cells in suspension culture (10,000 cells/ml RPMI-1640 growth medium) were incubated with serial dilutions of compound at 37°C for 48 hours and then pulsed labelled with 1 microCi of ³H-Tdr (SA = 20 Ci/mmole) for 8 hours at 37°C to measure DNA synthesis. Data are given in Table 4 below.

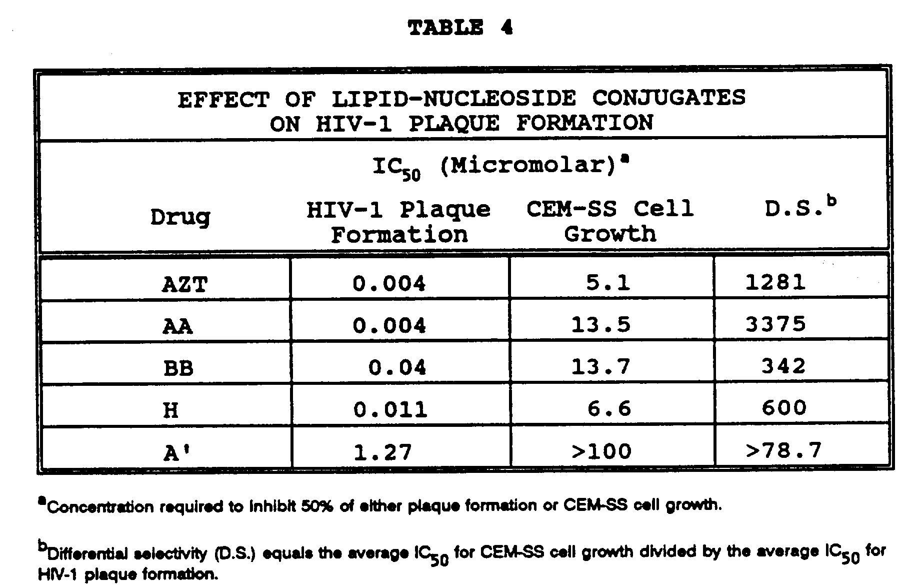

The foregoing examples are illustrative of the present invention, and are not to be construed as limiting thereof. For example, those skilled in the art will appreciate that minor changes can be made in the compounds disclosed herein which will not significantly adversely affect the activity and usefulness thereof. Accordingly, the invention is defined by the following claims.

## Claims

1. A lipid-nucleoside covalent conjugate or a salts thereof having the formula: 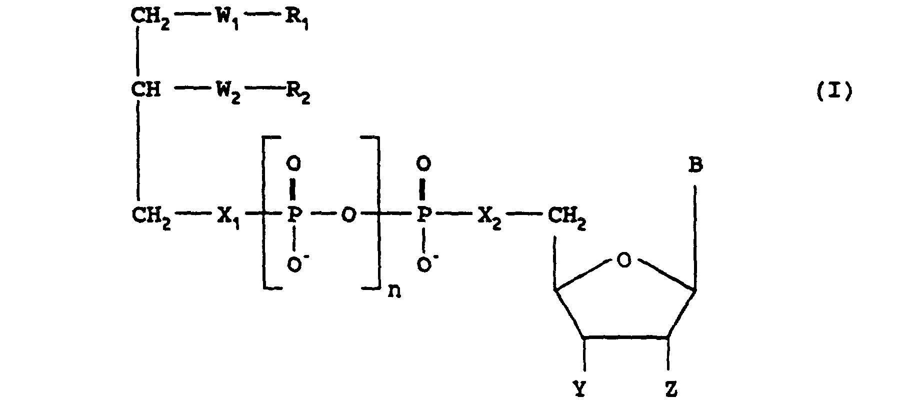 wherein:
R₁ is C10-C20 saturated or unsaturated alkyl containing not more than three double bonds;
R₂ is H or C1-C20 saturated or unsaturated alkyl containing not more than three double bonds; W₁ is S, O, NHC(=O), or NH;
W₂ is S, O, NHC(=O), OC(=O), NH, or a covalent bond;
n is zero or one.
X₁ and X₂ are each independently oxygen or a covalent bond, subject to the proviso that when n is zero, then at least either X₁ or X₂ is O;
Y is H, F, or N₃; Z is H or F; or Y and Z together are a covalent bond; and
B is selected from the group consisting of adenine, thymine, cytosine, guanine, hypoxanthine, uracil, 5-fluoro-cytosine, 2-fluoro-adenine, 2-chloro-adenine, 2-bromo-adenine, and 2-amino-adenine.

2. A lipid-nucleoside conjugate according to claim 1, wherein R₁ is C16-C18 linear alkyl containing not more than one double bond.

3. A lipid-nucleoside conjugate according to claim 1, wherein R₂ is H or C1-C3 alkyl.

4. A lipid-nucleoside conjugate according to claim 1, wherein W₁ is NHC(=O).

5. A lipid-nucleoside conjugate according to claim 1, wherein W₁ is NH.

6. A lipid-nucleoside conjugate according to claim 1, wherein W₂ is O.

7. A lipid-nucleoside conjugate according to claim 1, wherein Y is H or N₃; Z is H or F; or Y and Z together are a covalent bond.

8. A lipid-nucleoside conjugate according to claim 1, wherein Y is N₃, Z is H, and B is thymine.

9. A lipid-nucleoside conjugate according to claim 1, wherein n is 1, X₁ is O, and X₂ is O.

10. A lipid-nucleoside conjugate according to claim 1, wherein n is 1, X₁ is a covalent bond, and X₂ is O.

11. A lipid-nucleoside conjugate according to claim 1, wherrein n is 1, X₁ is O, and X₂ is a covalent bond.

12. A lipid-nucleoside conjugate according to claim 1, wherein n is 1, X₁ is a covalent bond, and X₂ is a covalent bond.

13. A lipid-nucleoside conjugate according to claim 1, wherein n is zero, X₁ is O, and X₂ is O.

14. A lipid-nucleoside conjugate according to claim 1, wherein n is zero, X₁ is a covalent bond, and X₂ is O.

15. A lipid-nucleoside conjugate according to claim 1, wherein n is zero, X₁ is O, and X₂ is a covalent bond.

16. A lipid-nucleoside conjugate according to claim 1 which is 3'-Azido-3'-deoxythymidine-5'-monophosphate-D,L-3-octadecanamido-2-ethoxypropane.

17. A lipid-nucleoside conjugate according to claim 1 which is 3'-Azido-3'-deoxythymidine-5'-monophosphate-D,L-3-hexadecyloxy-2-ethoxypropane.

18. A lipid-nucleoside conjugate according to claim 1 which is 3'-azido-3'-deoxythymidine.5'-monophosphate-D,L-3-hexadecylthio-2-methoxypropane.

19. A lipid-nucleoside conjugate according to claim 1 which is 2',3'-dideoxynosine-5'-monophosphate-D,L-3-octadecanamido-2-ethoxypropane.

20. A lipid-nucleoside conjugate according to claim 1 which is 3'-Azido-3'-deoxythymidine-5'-phosphono-D,L-3-hexadecyloxy-2-methoxypropane.

21. A lipid-nucleoside conjugate according to claim 1 which is 3'-azido-3'-deoxythymidine-5'-monophosphate-D,L-3-octadecanamido-2-hexadecyloxypropane.

22. A lipid-nucleoside conjugate according to any previous claim, wherein an HIV-I combating amount of the lipid-nucleoside conjugate is in combination with a pharmaceutically acceptable carrier.

23. A lipid-nucleoside conjugate or a salt thereof having the formula: 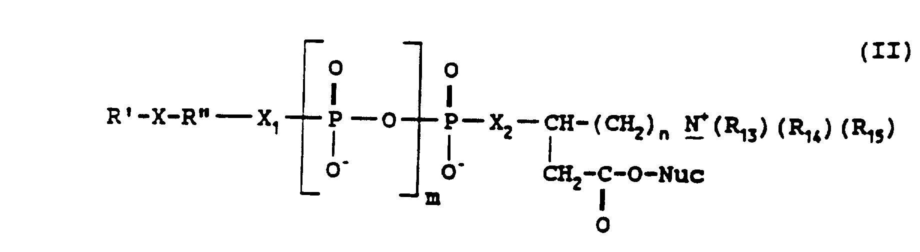 wherein:
X is S, O, NHC(=O), OC(=O), or NH;
R' is linear or branched, saturated or unsaturated C10-C20 alkyl containing not more than four double bonds, linear or branched, saturated or unsaturated C10-C20 acyl containing not more than four double bonds, phenyl, or naphthyl;
R" is C5 to C6 cycloalkylene, or a straight-chained or branched, saturated or unsaturated aliphatic hydrocarbon chain containing 2-8 carbon atom, which is unsubstituted or substituted one or more times by hydroxyl, phenyl, C1-C20 acyloxy, C1-C20 alkylthio, C1-C20 acylated amino, C1-C20 alkyl, or by C1-C20 alkoxy which is unsubstituted or is substituted by phenyl or C1-C5 alkoxy;
m is zero or one;
X₁ and X₂ are each independently oxygen or a covalent bond, subject to the proviso that when m is zero, then at least either X₁ or X₂ is O;
n is 1 to 3:
R₁₃, R₁₄, and R₁₅ are each independently either hydrogen or methyl;
Nuc is: 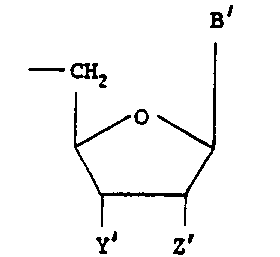
wherein:
Y' is H, F, or N₃; Z' is H or F; or Y' and Z' together are a covalent bond; and
B' is selected from the group consisting of adenine, thymine, cytosine, guanine, hypoxanthine, uracil, 5-fluoro-cytosine, 2-fluoro-adenine, 2-chloro-adenine, 2-bromo-adenine, and 2-amino-adenine.

24. A lipid-nucleoside conjugate according to claim 23 above, wherein X is NHC(=O).

25. A lipid-nucleoside conjugate according to claim 23 above, wherein R' is C14-C20 linear saturated or unsaturated alkyl containing not more than three double bonds.

26. A lipid-nucleoside conjugate according to claim 23 above, wherein R' is C16-C18 linear alkyl containing not more than one double bond.

27. A lipid-nucleoside conjugate according to claim 23 above, wherein R" is C2-C4 linear alkyl which is unsubstituted or is substituted one or two times by hydroxyl, phenyl, C1-C20 acyloxy, C1-C20 alkylthio, C1-C20 acylated amino or by C1-C20 alkoxy which is unsubstituted or is substituted by phenyl or C1-C5 alkoxy.

28. A lipid-nucleoside conjugate according to claim 23 above, wherein R" is linear C2-C4 alkyl which is unsubstituted or substituted one or two times by hydroxyl, phenyl, C1-C20 acyloxy, C1-C20 alkylthio, C1-C20 acylated amino or by C1-C20 alkoxy which is unsubstituted or is substituted by phenyl or C1-C5 alkoxy.

29. A lipid-nucleoside conjugate according to claim 23 above, wherein n is 1.

30. A lipid-nucleoside conjugate according to claim 23 above, wherein R₁₃, R₁₄, and R₁₅ are methyl.

31. A lipid-nucleoside conjugate according to claim 23 above, wherein Y' is H or N₃; Z' is H; or Y' and Z' together are a covalent bond.

32. A lipid-nucleoside conjugate according to claim 23 above, wherein Y' is H or N₃ and Z' is H.

33. A lipid-nucleoside conjugate according to claim 23, comprising 3'-Azido-3'-deoxythimidine-5'-butyrate-γ-N,N,N-trimethyl-ammonium-β-(1-phospho-2-ethoxy-3-hexadecyloxypropane).

34. A lipid-nucleoside conjugate according to claim 23, comprising 3'-Azido-3'-deoxthymidine-5'-butyrate-γ-N,N,N-trimethyl-ammonium-β-(1-phospho-2-ethoxy-3-octadecanamidopropane).

35. A lipid-nucleoside conjugate according to claims 23-34, wherein an HIV-I combating amount of the lipid-nucleoside conjugate is in combination with a pharmaceutically acceptable carrier.

## Patentansprüche

1. Kovalentes Lipid-Nucleosid-Konjugat oder ein Salz davon, mit der Formel: 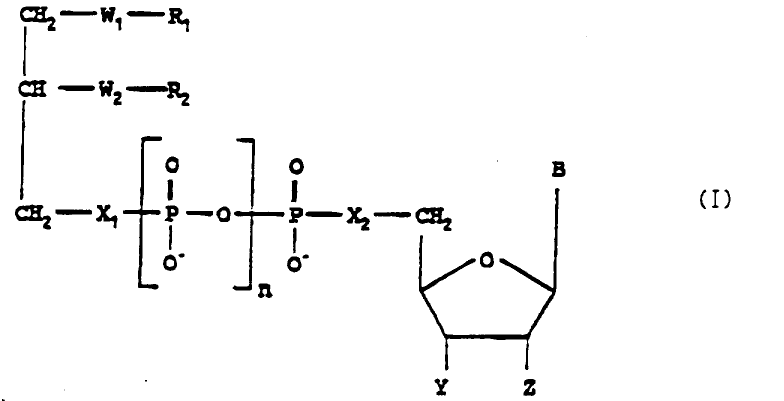 wobei:
R₁ ein gesättigter oder ungesättigter C₁₀-C₂₀-Alkylrest ist, der höchstens drei Doppelbindungen enthält;
R₂ gleich H oder ein gesättigter oder ungesättigter C₁-C₂₀-Alkylrest ist, der höchstens drei Doppelbindungen enthält;
W₁ gleich S, O, NHC(=O) oder NH ist;
W₂ gleich S, O, NHC(=O), OC(=O), NH oder eine kovalente Bindung ist;
n gleich null oder eins ist;
X₁ und X₂ unabhängig voneinander jeweils Sauerstoff oder eine kovalente Bindung bedeuten, vorausgesetzt, daß bei n gleich null von X₁ oder X₂ mindestens eines gleich O ist;
Y gleich H, F oder N₃; Z gleich H oder F ist; oder Y und Z zusammen eine kovalente Bindung sind; und
B aus der Gruppe ausgewahlt ist, die aus Adenin, Thymin, Cytosin, Guanin, Hypoxanthin, Uracil, 5-Fluorcytosin, 2-Fluoradenin, 2-Chloradenin, 2-Bromadenin und 2-Aminoadenin besteht.

2. Lipid-Nucleosid-Konjugat nach Anspruch 1, wobei R₁ ein linearer C₁₆-C₁₈-Alkylrest ist, der höchstens eine Doppelbindung enthält.

3. Lipid-Nucleosid-Konjugat nach Anspruch 1, wobei R₂ gleich H oder ein C₁-C₃-Alkylrest ist.

4. Lipid-Nucleosid-Konjugat nach Anspruch 1, wobei W₁ gleich NHC(=O) ist.

5. Lipid-Nucleosid-Konjugat nach Anspruch 1, wobei W₁ gleich NH ist.

6. Lipid-Nucleosid-Konjugat nach Anspruch 1, wobei W₂ gleich O ist.

7. Lipid-Nucleosid-Konjugat nach Anspruch 1, wobei Y gleich H oder N₃; Z gleich H oder F ist; oder Y und Z zusammen eine kovalente Bindung sind.

8. Lipid-Nucleosid-Konjugat nach Anspruch 1, wobei Y gleich N₃, Z gleich H ist und B Thymin bedeutet.

9. Lipid-Nucleosid-Konjugat nach Anspruch 1, wobei n gleich 1, X₁ gleich O und X₂ gleich O ist.

10. Lipid-Nucleosid-Konjugat nach Anspruch 1, wobei n gleich 1, X₁ eine kovalente Bindung und X₂ gleich O ist.

11. Lipid-Nucleosid-Konjugat nach Anspruch 1, wobei n gleich 1, X₁ gleich O und X₂ eine kovalente Bindung ist.

12. Lipid-Nucleosid-Konjugat nach Anspruch 1, wobei n gleich 1, X₁ eine kovalente Bindung und X₂ eine kovalente Bindung ist.

13. Lipid-Nucleosid-Konjugat nach Anspruch 1, wobei n gleich null, X₁ gleich O und X₂ gleich O ist.

14. Lipid-Nucleosid-Konjugat nach Anspruch 1, wobei n gleich null, X₁ eine kovalente Bindung und X₂ gleich O ist.

15. Lipid-Nucleosid-Konjugat nach Anspruch 1, wobei n gleich null, X₁ gleich O und X₂ eine kovalente Bindung ist.

16. Lipid-Nucleosid-Konjugat nach Anspruch 1, das die Verbindung 3'-Azido-3'-desoxythymidin-5'-monophosohat-D,L-3-octadecanamido-2-ethoxypropan ist.

17. Lipid-Nucleosid-Konjugat nach Anspruch 1, das die Verbindung 3'-Azido-3'-desoxythymidin-5'-monophosphat-D,L-3-hexadecyloxy-2-ethoxypropan ist.

18. Lipid-Nucleosid-Konjugat nach Anspruch 1, das die Verbindung 3'-Azido-3'-desoxythymidin-5'-monophosphat-D,L-3-hexadecylthio-2-methoxypropan ist.

19. Lipid-Nucleosid-Konjugat nach Anspruch 1, das die Verbindung 2',3'-Didesoxylnosin-5'-monophosphat-D,L-3-octadecanamido-2-ethoxypropan ist.

20. Lipid-Nucleosid-Konjugat nach Anspruch 1, das die Verbindung 3'-Azido-3'-desoxythymidin-5'-phosphon-D,L-3-hexadecyloxy-2-methoxypropan ist.

21. Lipid-Nucleosid-Konjugat nach Anspruch 1, das die Verbindung 3'-Azido-3'-desoxythymidin-5'-monophosphat-D,L-3-octadecanamido-2-hexadecyloxypropan ist.

22. Lipid-Nucleosid-Konjugat nach einem der vorstehenden Ansprüche, wobei eine für die HIV-I-Bekämpfung wirksame Menge des Lipid-Nucleosid-Konjugats mit einem pharmazeutisch annehmbaren Träger kombiniert wird.

23. Lipid-Nucleosid-Konjugat oder ein Salz davon, mit der Formel: 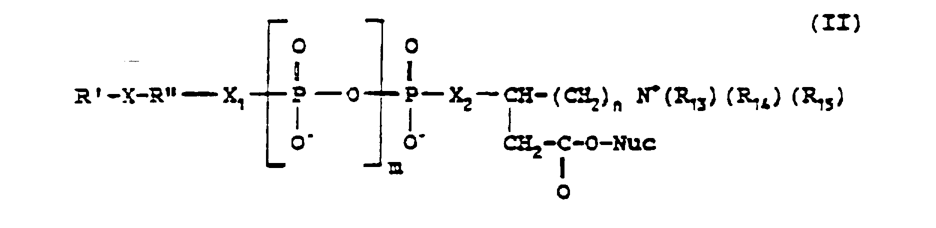 wobei:
X gleich S, O, NHC(=O), OC(=O) oder NH ist;
R' ein linearer oder verzweigter, gesättigter oder ungesättigter C₁₀-C₂₀-Alkylrest mit höchstens vier Doppelbindungen, ein linearer oder verzweigter, gesättigter oder ungesättigter C₁₀-C₂₀-Acylrest mit höchstens vier Doppelbindungen, ein Phenyl- oder Naphthylrest ist;
R" ein C₅- bis C₆-Cycloalkylen oder eine geradkettige oder verzweigte, gesättigte oder ungesättigte aliphatische Kohlenwasserstoffkette mit 2-8 Kohlenstoffatomen ist, die nichtsubstituiert oder ein- oder mehrfach mit Hydroxyl-, Phenyl-, C₁-C₂₀-Acyloxy-, C₁-C₂₀-Alkylthio-, acylierten C₁-C₂₀-Amino-, C₁-C₂₀-Alkylresten oder mit nichtsubstituierten oder phenyl- oder C₁-C₅-alkoxy-substituierten C₁-C₂₀-Alkoxyresten substituiert ist;
m gleich null oder eins ist;
X₁ und X₂ unabhängig voneinander jeweils Sauerstoff oder eine kovalente Bindung sind, vorausgesetzt, daß bei m gleich null von X₁ oder X₂ mindestens eines gleich O ist;
n gleich 1 bis 3 ist;
R₁₃, R₁₄ und R₁₅ unabhangig voneinander jeweils entweder Wasserstoff oder Methyl sind;
Nuc durch 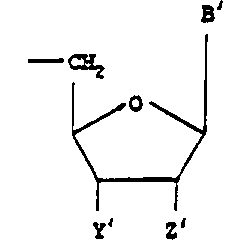
gegeben ist, wobei:
Y' gleich H, F oder N₃; Z' gleich H oder F ist; oder Y' und Z' zusammen eine kovalente Bindung sind; und
B' aus der Gruppe ausgewählt ist, die aus Adenin, Thymin, Cytosin, Guanin, Hypoxanthin, Uracil, 5-Fluorcytosin, 2-Fluoradenin, 2-Chloradenin, 2-Bromadenin und 2-Aminoadenin besteht.

24. Lipid-Nucleosid-Konjugat nach Anspruch 23, wobei X gleich NHC(=O) ist.

25. Lipid-Nucleosid-Konjugat nach Anspruch 23, wobei R' ein linearer gesättigter oder ungesättigter C₁₄-C₂₀-Alkylrest ist, der höchstens drei Doppelbindungen enthält.

26. Lipid-Nucleosid-Konjugat nach Anspruch 23, wobei R' ein linearer C₁₆-C₁₈-Alkylrest ist, der höchstens eine Doppelbindung enthält.

27. Lipid-Nucleosid-Konjugat nach Anspruch 23, wobei R" ein linearer C₂-C₄-Alkylrest ist, der nichtsubstituiert oder ein- oder zweifach mit Hydroxyl-, Phenyl, C₁-C₂₀-Acyloxy-, C₁-C₂₀-Alkylthio-, acylierten C₁-C₂₀-Aminoresten oder mit nichtsubstituierten oder phenyl- oder C₁-C₅-alkoxy-substituierten C₁-C₂₀-Alkoxyresten substituiert ist.

28. Lipid-Nucleosid-Konjugat nach Anspruch 23, wobei R" ein linearer C₂-C₄-Alkylrest ist, der nichtsubstituiert oder ein- oder zweifach mit Hydroxyl-, Phenyl, C₁-C₂₀-Acyloxy-, C₁-C₂₀-Alkylthio-, acylierten C₁-C₂₀-Aminoresten oder mit nichtsubstituierten oder phenyl- oder C₁-C₅-alkoxy-substituierten C₁-C₂₀-Alkoxyresten substituiert ist.

29. Lipid-Nucleosid-Konjugat nach Anspruch 23, wobei n gleich 1 ist.

30. Lipid-Nucleosid-Konjugat nach Anspruch 23, wobei R₁₃, R₁₄ und R₁₅ Methyl sind.

31. Lipid-Nucleosid-Konjugat nach Anspruch 23, wobei, Y' gleich H oder N₃; Z' gleich H ist; oder Y' und Z' zusammen eine kovalente Bindung sind.

32. Lipid-Nucleosid-Konjugat nach Anspruch 23, wobei Y' gleich H oder N₃ und Z' gleich H ist.

33. Lipid-Nucleosid-Konjugat nach Anspruch 23, das die Verbindung 3'-Azido-3'-desoxythymidin-5'-butyrat-γ-N,N,N-trimethylammonium-β-(1-phospho-2-ethoxy-3-hexadecyloxypropan) aufweist.

34. Lipid-Nucleosid-Konjugat nach Anspruch 23, das die Verbindung 3'-Azido-3'-desoxythymidin-5'-butyrat-γ-N,N,N-trimethylammonium-β-(1-phospho-2-ethoxy-3-octadecanamidopropan) aufweist.

35. Lipid-Nucleosid-Konjugat nach Anspruchen 23 bis 34, wobei eine für die HIV-I-Bekämpfung wirksame Menge des Lipid-Nucleosid-Konjugats mit einem pharmazeutisch annehmbaren Träger kombiniert wird.

## Revendications

1. Conjugué covalent de lipides-nucléosides ou un sel correspondant, ayant la formule: 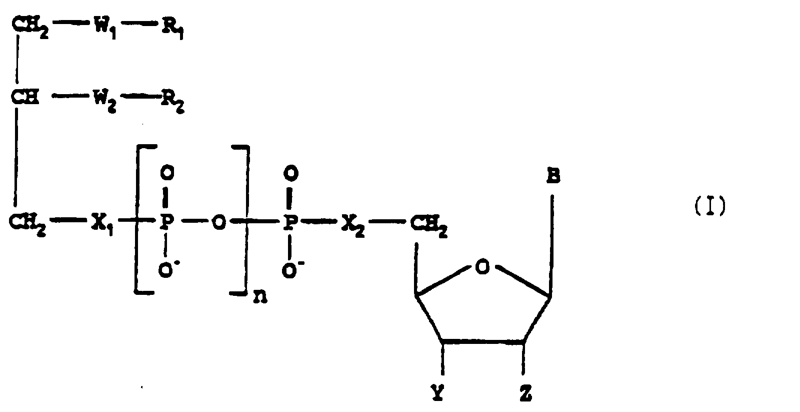 où:
R₁ est un alkyle saturé ou insaturé en C₁₀-C₂₀ ne contenant pas plus de trois liaisons doubles;
R₂ est de l'hydrogène ou un alkyle saturé ou insaturé en C₁-C₂₀ ne contenant pas plus de trois liaisons doubles; W₁ est du S, de l'oxygène, du NHC(=O) ou du NH:
W₂ est du S, de l'oxygène, du NHC(=O), du OC(=O), du NH ou une liaison covalente:
n correspond à zéro ou à un.
X₁ et X₂ sont chacun indépendamment de l'oxygène ou une liaison covalente, à condition que, lorsque n correspond à zéro. au moins un de X₁ ou de X₂ est de l'oxygène;
Y est de l'hydrogène, du F, ou du N₃; Z est de l'hydrogène ou du F; ou Y et Z constituent ensemble une liaison covalente; et
B est sélectionné dans le groupe constitué d'adénine, de thymine, de cytosine, de guanine, de hypoxanthine, d'uracile, de 5-fluoro-cytosine, de 2-fluoro-adénine, de 2-chlore-adénine, de 2-bromo-adénine et de 2-amino-adénine.

2. Conjugué de lipides-nucléosides selon la revendication 1, dans lequel ^{R}₁ est un alkyle linéaire en C₁₆-C₁₈ ne contenant pas plus d'une liaison double.

3. Conjugué de lipides-nucléosides selon la revendication 1, dans lequel R₂ est de l'hydrogène ou un alkyle en C₁-C₃.

4. Conjugué de lipides-nucléosides selon la revendication 1, dans lequel W₁ est du NHC(=O).

5. Conjugué de lipides-nucléosides selon la revendication 1, dans lequel W₁ est du NH.

6. Conjugué de lipides-nucléosides selon la revendication 1, dans lequel W₂ est de l'oxygène.

7. Conjugué de lipides-nucléosides selon la revendication 1, dans lequel Y est de l'hydrogène ou du N₃; Z est de l'hydrogène ou du F; ou Y et Z constituent ensemble une liaison covalente.

8. Conjugué de lipides-nucléosides selon la revendication 1, dans lequel Y est du N₃, Z est de l'hydrogène, et B est de la thymine.

9. Conjugué de lipides-nucléosides selon la revendication 1, dans lequel n correspond à 1, X₁ est de l'oxygène et X₂ est de l'oxygène.

10. Conjugué de lipides-nucléosides selon la revendication 1, dans lequel n correspond à 1, X₁ est une liaison covalente et X₂ est de l'oxygène.

11. Conjugué de lipides-nucléosides selon la revendication 1, dans lequel n correspond à 1, X₁ est de l'oxygène et X₂ est une liaison covalente.

12. Conjugué de lipides-nucléosides selon la revendication 1, dans lequel n correspond à 1, X₁ est une liaison covalente et X₂ est une liaison covalente.

13. Conjugué de lipides-nucléosides selon la revendication 1, dans lequel n correspond à zéro, X₁ est de l'oxygène et X₂ est de l'oxygène.

14. Conjugué de lipides-nucléosides selon la revendication 1, dans lequel n correspond à zéro, X₁ est une liaison covalente et X₂ est de l'oxygène.

15. Conjugué de lipides-nucléosides selon la revendication 1, dans lequel n correspond à zéro, X₁ est de l'oxygène et X₂ est une liaison covalente.

16. Conjugué de lipides-nucléosides selon la revendication 1, qui est du 3'-azido-3'-désoxythymidine-5'-monophosphate-D,L-3-octadécanamido-2-éthoxypropane.

17. Conjugué de lipides-nucléosides selon la revendication 1, qui est du 3'-azido-3'-désoxythymidine-5'-monophosphate-D,L-3-hexadécyloxy-2-éthoxypropane.

18. Conjugué de lipides-nucléosides selon la revendication 1, qui est du 3'-azido-3'-désoxythymidine-5'-monophosphate-D,L-3-hexadécylthio-2-méthoxypropane.

19. Conjugué de lipides-nucléosides selon la revendication 1, qui est du 2',3'-didesoxynosine-5'-monophosphate-D,L-3-octadécanamido-2-éthoxypropane.

20. Conjugué de lipides-nucléosides selon la revendication 1, qui est du 3'-azido-3'-désoxythymidine-5'-phosphono-D,L-3-hexadécyloxy-2-méthoxypropane.

21. Conjugué de lipides-nucléosides selon la revendication 1, qui est du 3'-azido-3'-désoxythymidine-5'-monophosphate-D,L-3-octadécanamido-2-hexadécyloxypropane.

22. Conjugué de lipides-nucléosides selon l'une quelconque des revendications précédentes, dans lequel une quantité anti-HIV-1 du conjugué de lipides-nucléosides est combinée avec un porteur pharmaceutiquement acceptable.

23. Conjugué de lipides-nucléosides ou un sel correspondant, ayant la formule: 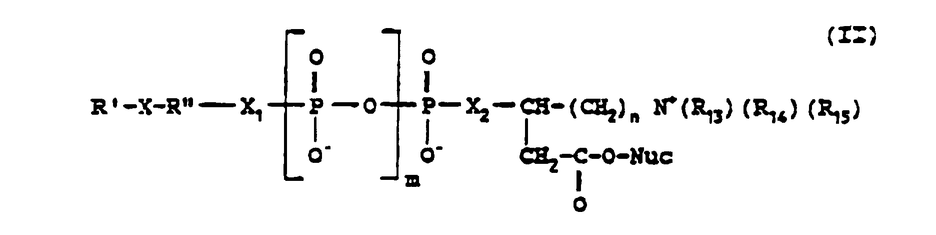 où:
X est du S, de l'oxygène, du NHC(=O), du OC(=O) ou du NH;
R' est un alkyle linéaire ou ramifié, saturé ou insaturé, en C₁₀-C₂₀, ne contenant pas plus de quatre liaisons doubles. un acyle linéaire ou ramifié, saturé ou insaturé, en C₁₀-C₂₀, ne contenant pas plus de quatre liaisons doubles, un phényle ou un naphtyle;
R" est un cycloalkylène en C₅ à C₆, ou une chaîne d'hydrocarbure aliphatique, linéaire ou ramifiée, saturée ou insaturée, contenant de 2 à 8 atomes de carbone, substituée ou non substituée une ou plusieurs fois par un hydroxyle, un phényle, un acyloxyde en C₁-C₂₀, un thioalkyle en C₁-C₂₀, une amine acylée en C₁-C₂₀, un alkyle en C₁-C₂₀, ou par un alkoxyde en C₁-C₂₀ non substitué ou substitué par du phényl ou un alkoxyde en C₁-C₅;
m correspond à zéro ou à un;
X₁ et X₂ sont chacun indépendamment de l'oxygène ou une liaison covalente, à condition que, lorsque m correspond à zéro, au moins un de X₁ ou de X₂ est de l'oxygène:
n correspond de 1 à 3;
R₁₃, R₁₄ et R₁₅ sont chacun indépendamment de l'hydrogène ou du méthyle;
Nuc correspond à: 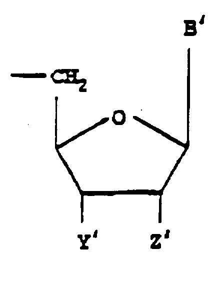 où:
Y' est de l'hydrogène, du F, ou du N₃; Z' est de l'hydrogène ou du F; ou Y' et Z' constituent ensemble une liaison covalente; et
B' est sélectionné dans le groupe constitué d'adénine, de thymine, de cytosine, de guanine, de hypoxanthine, d'uracile, de 5-fluoro-cytosine, de 2-fluoro-adénine, de 2-chloro-adénine, de 2-bromo-adénine et de 2-amino-adénine.

24. Conjugué de lipides-nucléosides selon la revendication 23, dans lequel X est du NHC(=O).

25. Conjugué de lipides-nucléosides selon la revendication 23, dans lequel R' est un alkyle linéaire, saturé ou insaturé, en C₁₄-C₂₀ ne contenant pas plus de trois liaisons doubles.

26. Conjugué de lipides-nucléosides selon la revendication 23, dans lequel R' est un alkyle linéaire en C₁₆-C₁₈, ne contenant pas plus d'une liaison double.

27. Conjugué de lipides-nucléosides selon la revendication 23, dans lequel R" est un alkyle linéaire en C₂-C₄, non substitué ou substitué une ou deux fois par un hydroxyle, un phényle, un acyloxyde en C₁-C₂₀, un thioalkyle en C₁-C₂₀, une amine acylée en C₁-C₂₀ ou par un alkoxyde en C₁-C₂₀ non substitué ou substitué par un phényle ou un alkoxyde en C₁-C₅.

28. Conjugué de lipides-nucléosides selon la revendication 23, dans lequel R" est un alkyle linéaire en C₂-C₄, non substitué ou substitué une ou deux fois par un hydroxyle, un phényle, un acyloxyde en C₁-C₂₀, un thioalkyle en C₁-C₂₀, une amine acylée en C₁-C₂₀ ou par un alkoxyde en C₁-C₂₀ non substitué ou substitué par un phényle ou un alkoxyde en C₁-C₅.

29. Conjugué de lipides-nucléosides selon la revendication 23, dans lequel n correspond à 1.

30. Conjugué de lipides-nucléosides selon la revendication 23, dans lequel R₁₃, R₁₄ et R₁₅ sont du méthyle.

31. Conjugué de lipides-nucléosides selon la revendication 23, dans lequel Y' est de l'hydrogène ou du N₃; Z' est de l'hydrogène: ou Y' et Z' constituent ensemble une liaison covalente.

32. Conjugué de lipides-nucléosides selon la revendication 23, dans lequel Y' est de l'hydrogène ou du N₃ et Z' est de l'hydrogène.

33. Conjugué de lipides-nucléosides selon la revendication 23, comprenant du 3'-azido-3'-désoxythymidine-5'-butyrate-γ-N,N,N-triméthyl-ammoniun-β-(1-phospho-2-éthoxy-3-hexadécyloxypropane).

34. Conjugué de lipides-nucléosides selon la revendication 23, comprenant du 3'-azido-3'-désoxythymidine-5'-butyrate-γ-N,N,N-triméthyl-ammonium-β-(1-phospho-2-éthoxy-3-octadécanamidopropane).

35. Conjugué de lipides-nucléosides selon les revendications 23 à 34, dans lequel une quantité anti-HIV-I du conjugué de lipides-nucléosides est combinée avec un porteur pharmaceutiquement acceptable.
